# EUROPEAN PATENT SPECIFICATION

(11) **EP 1 863 934 B1**
(45) Date of publication and mention of the grant of the patent: **04.11.2009**
(21) Application number: 06739112.8
(22) Date of filing: 20.03.2006
(51) Int. Cl.: C12Q 1/68

(54) **UNIQUE SEQUENCE HYBRIDIZATION PROBES (USP)**
EINZELSEQUENZHYBRIDISIERUNGSSONDEN
SONDES D'HYBRIDATION UNISÉQUENTIELLES

(30) Priority: 29.03.2005 US 666000 P; 22.04.2005 US 112926
(43) Date of publication of application: 12.12.2007
(73) Proprietor: Exagen Diagnostics, Inc., Albuquerque, NM 87106 (US)
(72) Inventor: DAVIS, Lisa, Albuquerque, NM 87106 (US); TANG, Lei, Albuquerque, NM 87122 (US)
(74) Representative: Zwicker, Jörk
(86) International application number: PCT/US2006/010195
(87) International publication number: WO 2006/104761

(56) References cited:
- WO-A-01/88089
- KNOLL JOAN H M ET AL: "Sequence-based, in situ detection of chromosomal abnormalities at high resolution." AMERICAN JOURNAL OF MEDICAL GENETICS, vol. 121A, no. 3, 1 September 2003 (2003-09-01), pages 245-257, XP002397525 ISSN: 0148-7299
- ROGAN PETER K ET AL: "Sequence-based design of single-copy genomic DNA probes for fluorescence in situ hybridization" GENOME RESEARCH, vol. 11, no. 6, June 2001 (2001-06), pages 1086-1094, XP002397526 ISSN: 1088-9051

## Description

### Cross Reference

This application claims priority to U.S. Provisional Patent Application Serial No. 60/666,000 filed March 29, 2005 and to U.S. Utility Patent Application 11/112,926 filed April 22, 2005,

### Field of the Invention

This invention relates generally to the fields of nucleic acids, detection, hybridization, diagnostics, and prognostics.

### Background

Nucleic acid detection generally involves hybridization via base-pair binding of nucleic acid probes to a nucleic acid target. Such detection assays find wide utility in the areas of basic and clinical research, as well as in a variety of diagnostic and prognostic applications. Nucleic acid detection can be carried out to detect a target nucleic acid in complex samples, such as genomic DNA, total RNA, chromosome spreads, cells, and tissue samples. Similarly, probes for the target of interest often contain both a unique sequence component, specific for the target, and one or more repetitive sequence DNA elements that are not specific for the target. If the repetitive sequences are not disabled, the probe reacts with multiple repeat-containing nucleic acids in the sample, and thus will not specifically react with the target nucleic acid. This problem is particularly acute with interspersed repeat sequences which are widely scattered throughout the genome, but also is present with tandem repeats clustered or contiguous on the DNA molecule.

Non-specific hybridization of repetitive sequences can be disabled in several ways. The most common method for disabling repetitive sequences is to block the repetitive sequence/sequences by pre-association with excess unlabeled repetitive sequence containing complimentary fragments such as COT-1 DNA, generically known as competitor nucleic acid or blocking nucleic acid. In its typical usage, the repetitive sequences in the probe are annealed to the complimentary unlabeled repetitive sequences in the competitor nucleic acid prior to target hybridization, a process routinely referred to as "pre-annealing." During the pre-annealing step, the labeled repetitive sequences of the probe and the unlabeled repetitive sequences of the competitor nucleic acid rapidly hybridize over the entire length of shared complementarity. Since only single stranded nucleic acid probe is able to hybridize to the target nucleic acid, the repetitive sequence in the probe is no longer available to hybridize to the target, leaving only the unique sequence component of the probe available for hybridization. Commercial application of this method requires preparation, or purchase from a commercial source, of large quantities of competitor nucleic acid which can be prohibitively time consuming and expensive.

Other techniques for disabling the repetitive sequence components of nucleic acid probes include those that call for additional manipulation steps of the probe itself prior to hybridization (see Craig et al., Hum Genet 1997 Sep;100(3-4):472-6; and Hozier et al., Cytogenet. Cell Genet 1998;83(1-2):60-3), which limit their applicability to commercial preparation of probe sets.

The use of probes for *in situ* hybridization has been disclosed in Knoll Joan H M et al: (2003), American Journal of Medical Genetics, vol. 121 A, no. 3, pages 245-257; in Rogan Peter K et al: (2001), Genome Research, vol. 11, no. 6, pages 1086-1094; and in WO 01/88089.

Attempts to use polymerase chain reaction ("PCR") to generate unique sequence probes from repetitive DNA-containing probes have met with some success; however, these methods required purification of appropriate sized PCR product by gel electrophoresis to remove single-stranded PCR extension products with flanking repetitive sequences. (Rogan et al., Genome Research 11:1086-1094 (2001); US 6,828,097) As a result, the method is not applicable to commercial scale production, or to the preparation, for example, of larger numbers of unique sequence probes for a large chromosomal region target nucleic acid.

Thus, improved methods for preparation of unique sequence probes are desirable, particularly for use in commercial production of such unique sequence probes.

### Summary of the Invention

The present invention provides methods for the preparation of unique sequence probes and their use. In one aspect, the invention provides methods
for preparing unique sequence probes comprising:
(a) identifying unique sequence elements contained in a nucleic acid probe, wherein the nucleic acid probe comprises at least 10 unique sequence elements and a plurality of repetitive sequence elements, and wherein the at least 10 sequence elements in the nucleic acid probe selectively bind to a nucleic acid target of interest;
(b) amplifying at least 10 unique sequence elements in the nucleic acid probe to generate at least 10 amplified unique sequence elements, wherein the amplifying comprises
   (i) contacting the nucleic acid probe with at least 10 primer sets for the at least 10 unique sequence elements under conditions to promote hybridization of the at least 10 primer sets to the nucleic acid probe, wherein each primer in the at least 10 primer sets has an annealing temperature of between 52° C and 65° C, and wherein the annealing temperature for all of the primers in the at least 10 primer sets are within 3° C of each other; and
   (ii) amplifying the at least 10 unique sequence elements defined by the at least 10 primer sets by polymerase chain reaction under conditions that comprise annealing the at least 10 primer sets to the nucleic acid probe at a temperature between 51° C and 65 ° C;
(c) subjecting those PCR reactions that resulted in amplification of the desired unique sequence element to a second round of PCR, using the same primer sets and PCR conditions; and
(d) pooling the amplified unique sequence elements to produce the unique sequence probe.

In a further aspect, the present invention provides methods for detecting a nucleic acid target of interest, comprising:
(a) generating a unique sequence probe according to the methods of the invention for the nucleic acid target of interest;
(b) contacting the unique sequence probe to a specimen to be tested under conditions to promote hybridization of the unique sequence probe to the nucleic acid target; and
(c) detecting hybridization complexes formed between the unique sequence probe and the nucleic acid target, wherein such hybridization complexes provide a measure of the nucleic acid target in the specimen.

### Brief Description of the Figures

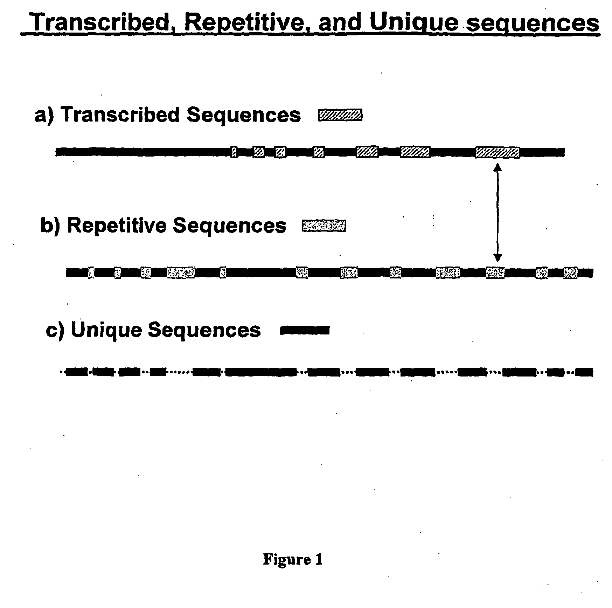

**Figure 1**. Schematic representation of a genome region showing the relationship between a) transcribed sequences, b) repetitive sequences, and c) unique sequences. Note that repetitive sequences are randomly dispersed, of varying lengths, and can occur between transcribes segments of a single gene and within transcribe sequences (indicated by the double arrow).

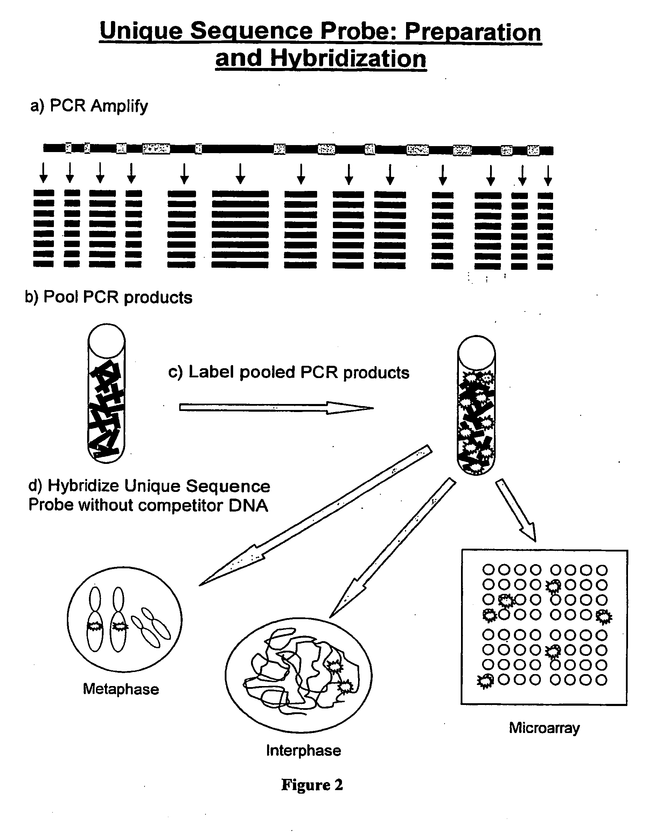

**Figure 2** is a diagram of a representative method for producing unique sequence probes according to the present invention. A) Each unique sequence segment of the genome regions is amplified with a unique pair of primers. (b) The PCR products are pooled, c) labeled with a fluorochrome, and (d) hybridized to complex targets including metaphase chromosome spreads, interphase cells, or DNA microarray chromosomes.
**Figure 3** is a table listing the primers used to make the SMARCE1 USP.
**Figure 4** is a table listing the primers used to make the PDCD6IP USP.
**Figure 5** is a table listing the primers used to make the CYP24 USP.
**Figure 6** is a table listing the primers used to make the NR1D1 USP.
**Figure 7** is a table listing the primers used to make the BIRC5 USP.

### Detailed Description of the Invention

Within this application, unless otherwise stated, the techniques utilized may be found in any of several well-known references such as: Molecular Cloning: A Laboratory Manual (Sambrook, et al., 1989, Cold Spring Harbor Laboratory Press), Gene Expression Technology (Methods in Enzymology, Vol. 185, edited by D. Goeddel, 1991. Academic Press, San Diego, CA), "Guide to Protein Purification" in Methods in Enzymology (M.P. Deutshcer, ed., (1990) Academic Press, Inc.); PCR Protocols: A Guide to Methods and Applications (Innis, et al. 1990. Academic Press, San Diego, CA), Culture of Animal Cells: A Manual of Basic Technique, 2nd Ed. (R.I. Freshney. 1987. Liss, Inc. New York, NY), Gene Transfer and Expression Protocols, pp. 109-128, ed. E.J. Murray, The Humana Press Inc., Clifton, N.J.), and the Ambion 1998 Catalog (Ambion, Austin, TX); Fish: A Practical Approach, Barbara G. Beatty, Sabine Mai, and Jeremy A. Squire, editors (Oxford University Press, 2002).

The present invention provides methods for the preparation of unique sequence probes. These methods circumvent the need for competitor DNA and for laborious post-amplification purification, and thus are especially useful for commercial preparation of unique sequence probes, although they can be used for any preparation of unique sequence probes.

In a first aspect, the invention provides methods
for preparing unique sequence probes comprising:
(a) identifying unique sequence elements contained in a nucleic acid probe, wherein the nucleic acid probe comprises at least 10 unique sequence elements and a plurality of repetitive sequence elements, and wherein the at least 10 sequence elements in the nucleic acid probe selectively bind to a nucleic acid target of interest;
(b) amplifying at least 10 unique sequence elements in the nucleic acid probe to generate at least 10 amplified unique sequence elements, wherein the amplifying comprises
   (i) contacting the nucleic acid probe with at least 10 primer sets for the at least 10 unique sequence elements under conditions to promote hybridization of the at least 10 primer sets to the nucleic acid probe, wherein each primer in the at least 10 primer sets has an annealing temperature of between 52° C and 65° C, and wherein the annealing temperature for all of the primers in the at least 10 primer sets are within 3° C of each other; and
   (ii) amplifying the at least 10 unique sequence elements defined by the at least 10 primer sets by polymerase chain reaction under conditions that comprise annealing the at least 10 primer sets to the nucleic acid probe at a temperature between 51° C and 65 ° C;
(c) subjecting those PCR reactions that resulted in amplification of the desired unique sequence element to a second round of PCR, using the same primer sets and PCR conditions; and
(d) pooling the amplified unique sequence elements to produce the unique sequence probe.

As used herein, a "nucleic acid probe" (or "probe") is any nucleic acid probe for a target of interest, wherein the nucleic acid probe contains a plurality of both unique sequence elements and repetitive sequence elements. Thus, the nucleic acid probe comprises both "unique" DNA sequences which are complementary to and specific for at least a portion of a target nucleic acid of interest and "repetitive" DNA sequences, which appear repeatedly in the genome of which the target nucleic acid is a part. Such nucleic acid probes can be derived from many different sources, including but not limited to chromosomal DNA, genomic DNA libraries, (including bacterial artificial chromosome (BAC) DNA libraries, yeast artificial chromosome (YAC) DNA libraries, P1 DNA libraries, lambda clone libraries, cosmid clone libraries, fosmid libraries, plasmid DNA libraries), and cDNA copies of expressed genes, cDNA clones isolated from cDNA or expression libraries, and polymerase chain reaction products prepared directly from genomic DNA. While the length of the nucleic acid probe is not critical to the invention, it is preferred that it is at least 2 kilobases ("kb") in length; in various preferred embodiments, the nucleic acid probe is at least 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 300, 350, 400, 450, 500, 550, 600, 650, 700, 750, 800, 850, 900, 950, 1000, 1500, 2000, 2500, 3000, 3500, 4000, 4500, or 5000 kb in length.

Prior to use in the methods of the invention, the nucleic acid probe is preferably double stranded. The nucleic acid probe can be free in solution or can be contained within a vector of some sort, including plasmids, viruses, and bacterial artificial chromosomes.

As used herein, the "nucleic acid target of interest" can be any nucleic acid target detectable by using the unique sequence probes according to the methods of the invention. In a preferred embodiment, the nucleic acid target of interest comprises a single copy gene, or a non-coding unique sequence segment of a gene, such as an intron, or the non-translated 3' or 5' region of a gene, or a unique intergenic segment in a genome. As used herein, a "single copy gene" is one that can be distinguished using standard DNA hybridization methods (ie: hybridization conditions and probe selection, such that only the desired target is detected under standard hybridization and wash conditions) from other genes in the genome in which the target nucleic acid is present. The nucleic acid target of interest can also comprise a member of a gene family, wherein at least some of the USPs share a region of homology with one or more members of the gene family, such that some or all members of the family are detected as unique entities, but still without cross-hybridization to unrelated regions of the genome due to the repetitive elements of the original probe.

As used herein, "unique sequence elements" are those portions of the nucleic acid probe that do not include a repetitive sequence element, but are bounded in the nucleic acid probe by repetitive sequence elements. In a preferred embodiment, the "unique sequence elements" can be used to selectively hybridize under standard hybridization conditions to a single copy gene, or any unique sequence region, in the genome of which the target nucleic acid is a part.

As used herein, "repetitive sequence elements" are those nucleic acid sequence portions of the nucleic acid probe that hybridize under standard hybridization conditions to repetitive sequences in the genome of which the target nucleic acid is a part, and include highly repetitive sequences, moderately repetitive sequences, tandem repeats (including but not limited to satellites, mini-satellites and micro-satellites), interspersed repeats (including, but not limited to ALUs, LINES and SINES), and palindromic repeats. Such repetitive sequence elements can be located near centromeres and telomeres, within heterogeneously staining regions (HSRS), distributed over a single chromosome, or throughout some or all chromosomes in the genome from which the target nucleic acid is derived. The presence of such repetitive sequence elements in a hybridization probe renders the probe non-specific if used as a whole in, for example, in situ hybridization assays on cells, tissue sections, or chromosome spreads.

The nucleic acid probes used in the method of the invention contain a plurality of unique sequence elements and repetitive elements; in preferred embodiments, the nucleic acid probe contains at least 15, 20, 25, 30, 35, 40, 45, 50, 55, 60, 65, 70, 75, 80, 85, 90, 95, 100, 200, 300, 400, 500, 600, 700, 800, 900, or 1000 unique sequence elements bounded by an approximately equal number of repetitive sequence elements.

As used herein, the term "plurality" means two or more.

As used herein, the phrase "identifying unique sequence elements" means determining the location of a plurality of unique sequence elements within the nucleic acid probe. This does not require the identification of all of the unique sequence elements in a given nucleic acid probe. For example, if a nucleic acid probe of interest contains 50 unique sequence elements, the "identifying" can comprise, for example, identifying 10 of the unique sequence elements. In various preferred embodiments, the identifying comprises identifying at least 10%, 20%, 30%, 40%, 50%, 60%, 70%, 80%, 90%, or 100% of the unique sequence elements in the nucleic acid probe. In various other preferred embodiments, the identifying comprises identifying 10, 15, 20, 25, 30, 35, 40, 45, 50, 55, 60, 65, 70, 75, 80, 85, 90, 95, 100, 200, 300, 400, 500, 600, 700, 800, 900, or 1000 unique sequence elements in the nucleic acid probe. It will be understood by those of skill in the art that such identification can be by any means known in the art, including by DNA sequence analysis and comparison to known repetitive sequence elements, review of DNA sequence information stored in various databases that contain sequence information for a nucleic acid probe of interest (for example, as discussed in the examples below), or by other computational methods. Such known-sequence based techniques provide the sequence from which PCR primers are designed and thus are preferred. It will also be understood that the unique sequence elements can be identified by inference, based on identification of the repetitive sequence elements in the nucleic acid probe. In one such example, repetitive sequences are identified by hybridization with a repetitive sequence probe and all elements that don't hybridize to it are considered as unique sequences. In this case, at least a portion of the unique sequence element DNA is preferably sequenced to define appropriate primers for amplification.

As used herein, the term "amplifying a plurality of unique sequence elements" means producing additional copies of two or more of the identified unique sequence elements, or portions thereof, in the nucleic acid probe; this amplification generates "amplified unique sequence elements." It will be understood by those of skill in the art that it not necessary to amplify each of the identified unique sequence elements in the nucleic acid probe. For example, if 50 unique sequence elements are identified in the nucleic acid probe, the "amplifying" can comprise, for example, amplifying 10 of the unique sequence elements. In various preferred embodiments, the amplifying comprises amplifying at least 10%, 20%, 30%, 40%, 50%, 60%, 70%, 80%, 90%, or 100% of the identified unique sequence elements in the nucleic acid probe. In various other preferred embodiments, the amplifying comprises amplifying 5, 10, 15, 20, 25, 30, 35, 40, 45, 50, 55, 60, 65, 70, 75, 80, 85, 90, 95, or 100, 200, 300, 400, 500, 600, 700, 800, 900, or 1000 of the identified unique sequence elements in the nucleic acid probe. It will be further understood by those of skill in the art that it is not necessary to amplify the entire portion of a given identified unique sequence element. Thus, all or a portion of a given identified unique sequence element may be amplified, based on polynucleotide probe design considerations (see below) and other experimental considerations within the knowledge of those of skill in the art.

As used herein, a "primer set" refers to a group of nucleic acid polynucleotide primers (2 or more primers; preferably 2) that are complementary to a given unique sequence element in the nucleic acid probe and can be used in a polymerase chain reaction ("PCR") to amplify a defined region of the given unique sequence element. In general, this requires at least two polynucleotide primers, one complementary to the 5' end of one strand of the nucleic acid probe and the other polynucleotide primer complementary to the 5' end of the other strand of the nucleic acid probe. There is no particular size constraint on the size of the amplified unique sequence element, other than labeling efficiency (where labeling the USP is desired) and the limitations of PCR. Thus, in a preferred embodiment, the length of the amplified unique sequence element should be at least 200 nucleotides, and up to any desired size. Those skilled in the art know how to optimize PCR conditions to achieve products of the required or desired size.

As will be understood by those of skill in the art, a plurality of such polynucleotide primer sets can be used to amplify a plurality of different unique sequence elements from the nucleic acid probe. While it is preferred that each primer set is used to amplify a different unique sequence element, multiple primer sets can also be used to amplify different regions of a given unique sequence element, as will be apparent to those of skill in the art.

Each polynucleotide primer in the plurality of primer sets has an annealing temperature of between 52°C and 65°C; preferably between 55°C and 63°C, and more preferably between 56°C and 61°C. In any of these embodiments, all of the polynucleotides in the various primer sets for a single nucleic acid probe have an annealing temperature within 0, 1, 2, or 3°C of each other.

By way of a non-limiting example, in preparing a USP for a NR1D1 BAC probe (see below) the annealing temperatures for each primer might range from 57-59°C, with an annealing temperature of 56°C being used during PCR in the NR1D1 USP probe preparation. Similarly, in preparing a USP for a CYP24 BAC probe, the annealing temperatures for each primer might range from 59-61°C, with an annealing temperature of 58°C being used in PCR in the CYP24 USP probe preparation.

As will be understood by those of skill in the art, the polynucleotide primer sequence should not be substantially self-complementary or complementary to the other member of the primer pair. Furthermore, the primers should not be substantially complementary to internal sites within the nucleic acid probe (other than the desired site); the primers should not form hairpin loops internally or with the nucleic acid probe or another polynucleotide primer in its primer set.

Many primer designs software are readily available and freely accessible on the internet (for example http://www.bioinformatics.vg). In one embodiment, primers can be designed and annealing temperatures determined using "FAST PCR" software (http://www.biocenter.helsinki.fi/bi/bare-1_html/oligos.htm.) Fast PCR software is applicable for many PCR applications including standard and long PCR, inverse PCR, and multiplex PCR. Because the program analyzes several sequences simultaneously (up to 1,000,000), primer design for the amplification of more than one product in a single reaction vessel is supported, including design for amplification in the same buffer and at the same annealing temperature. All primers are analyzed for melting temperature using the nearest neighbor thermodynamic theory with unified dS, dH and dG parameters to ensure accurate optimal annealing temperature prediction. The primer design program specifically identifies and eliminates primer sequence that might create pitfalls such as primer-dimer formation, self-complementarity, too low-melting temperature of primers, incorrect internal stability profile, false priming, and primer duplication site control. The program is also designed to accommodate one's personal database by local alignment and other publicly available bioinformatics tools are included. Those of skill in the art will recognize that other such programs are available for primer design.

Alternatively, polynucleotide primers for use in the present invention can be designed by those of skill in the art without the use of specific software, based on the knowledge of the relevant unique sequence elements, and teachings provided herein.

Thus, the length of the polynucleotide primer can vary (as disclosed in the examples that follow), so long as the annealing temperature of the primer has an annealing temperature of between 52°C and 65°C, and so long as all of the primers in the plurality of primer sets have annealing temperatures within 0, 1, 2, or 3°C of each other. Use of these conditions permits the production of unique sequence probes that circumvent the need for competitor DNA and for laborious post-amplification purification.

Synthetic polynucleotides can be prepared by a variety of solution or solid phase methods known in the art. For example, detailed descriptions of the procedures for solid phase synthesis of polynucleotide by phosphite-triester, phosphotriester, and H-phosphonate chemistries are widely available. (See, for example, US 6,664,057 and references disclosed therein). Methods to purify polynucleotides include native acrylamide gel electrophoresis, and anion-exchange HPLC, as described in Pearson (1983) J. Chrom. 255:137-149. The sequence of the synthetic polynucleotides can be verified using standard DNA sequencing methods, and are readily available from many commercial sites (such as Integrated DNA Technologies (Coralville, Iowa).

The term "polynucleotide" as used herein with respect to each aspect and embodiment of the invention refers to DNA or RNA, preferably DNA, in either single- or double-stranded form, preferably single stranded. The term "polynucleotide" encompasses nucleic acids containing known analogues of natural nucleotides which have similar or improved binding properties, for the purposes desired, as the reference polynucleotide. The term also encompasses nucleic-acid-like structures with synthetic backbones. DNA backbone analogues provided by the invention include phosphodiester, phosphorothioate, phosphorodithioate, methylphosphonate, phosphoramidate, alkyl phosphotriester, sulfamate, 3'-thioacetal, methylene(methylimino), 3'-N-carbamate, morpholino carbamate, and peptide nucleic acids (PNAs), methylphosphonate linkages or alternating methylphosphonate and phosphodiester linkages (Strauss-Soukup (1997) Biochemistry 36:8692-8698), and benzylphosphonate linkages, as discussed in US 6,664,057; see also Oligonucleotides and Analogues, a Practical Approach, edited by F. Eckstein, IRL Press at Oxford University Press (1991); Antisense Strategies, Annals of the New York Academy of Sciences, Volume 600, Eds. Baserga and Denhardt (NYAS 1992); Milligan (1993) J. Med. Chem. 36:1923-1937; Antisense Research and Applications (1993, CRC Press).

In a preferred embodiment, each of the primer sets is used to amplify its relevant unique sequence element from the nucleic acid probe in a separate PCR reaction. In a most preferred embodiment, the nucleic acid probes are amplified in a multiwell format with each primer pair pre-aliquoted to a single well of the multiwell plate. Alternatively, multiple primer sets can be present in individual PCR reactions. For example, a given unique sequence element can be amplified using more than 1 primer set, such as 2 or more primer sets that are used to amplify different portions of the unique sequence element. In a further alternative, 2 or more primer sets can be used to amplify 2 or more different unique sequence elements. In these embodiments, it is preferred that between 2 and 10 different primer pairs are used to amplify 1 or more unique sequence element in a single PCR reaction; more preferable between 2 and 7 different PCR primers, even more preferably between 2 and 5 different PCR primers, and even more preferably between 2 and 3 different PCR primers.

It is further preferred that the PCR reactions are carried out simultaneously, for example in a microplate adapted for use in PCR. Those of skill in the art are well-versed in such techniques. Exemplary manufacturers of such plates and PCR devices include Applied BioSystems, Perkin Elmer, and MJ Devices.

PCR conditions to promote amplification of the unique sequence elements can be any of those that are standard in the art, with the proviso that the conditions comprise annealing of the plurality of the primer sets to the nucleic acid probe at a temperature between 51°C and 65°C. In a preferred embodiment, the annealing step of the PCR reaction is conducted at a temperature approximately 1°C below that of the annealing temperature of the polynucleotide primers with the lowest annealing temperature in the primer set.

Determination of an appropriate amount of nucleic acid probe used in a PCR reaction with a single primer set is within the level of those of skill in the art. For example, a BAC clone of approximately 150-200 kb is preferably used at between about 0.01 and 10.0 ng/µl; more preferably between about 0.05 and about 5 ng/µl; even more preferably between about 0.1 and 1.0 ng/µl; and most preferably between about 0.3 and about 0.5 ng/µl.

Determination of an appropriate amount of a given polynucleotide primer used in a PCR reaction is within the level of those of skill in the art. In a preferred embodiment, the polynucleotide primer concentration ranges from about 0.1 µM to about 20 µM; more preferably between about 0.5 µM and 10 µM; most preferably between about 1 µM and 2.0 µM.

The PCR reaction mixture will further comprise standard components, including but not limited to DNA polymerase (such as Taq^{™} DNA Polymerase) present at, for example, 5-50 units/ml), an aqueous buffer medium including a source of cations (for example, MgCl₂, present at between 0.5 and 5 mM), nucleotides (for example, dATP, dGTP, dCTP, and dTTP, each present at between about 100 µM and about 500 µM) and a buffering agent (such as Tris). The pH of the reaction mixture is preferably between 6.5 and 9.0, more preferably between 7.0 and 8.5. It will be understood by those of skill in the art that other components can also be present in the PCR mix, including but not limited to melting point reducing agents (such as formamide).

The resulting PCR reaction mixture is then put through multiple cycles of denaturation, annealing, and polymerization. The denaturing steps, polymerization steps, and determination of number of reaction cycles to employ can be carried out using any such techniques in the art. Exemplary thermal cyclers for use with the methods of the invention are described in U.S. Pat. Nos. 5,612,473; and 5,602,756.

In exemplary embodiments, an initial denaturation step is optionally carried out at between about 90°C and 100°C; more preferably between about 92°C and about 98°C; more preferably between about 94°C and about 96°C. This initial optional step is carried out for between about 1-10 minutes; more preferably between about 2-8 minutes; and most preferably between about 4-6 minutes.

Following this optional initial denaturation step, exemplary, non-limiting reaction cycles (20-40 cycles; preferably 25-35 cycles; more preferably 28-32 cycles) are as follows, where the reaction is held at the temperatures specified below for between 5-120 seconds, more preferably between 10-90 seconds; more preferably between 15-60 seconds; and even more preferably between 15-30 seconds:
1- Denaturation: the temperature is held at between about 90°C and 98°C; more preferably between about 92°C and about 98°C; more preferably between about 94°C and about 96°C;
2- Annealing: the temperature is held at between about 51°C and 65°C; more preferably between about 53°C and about 63°C; more preferably between about 54°C and about 61°C ; and more preferably between about 55°C and about 59°C.
3- Extension step: the temperature is held at between about 66°C and 78°C; more preferably between about 68°C and about 76°C; more preferably between about 70°C and about 74°C; more preferably at 72°C.

As will be understood by those of skill in the art, the time at each step is dependent in part of the thickness of the wall of the PCR tube and the instrument itself.

After amplification, the amplified unique sequence elements are pooled to produce the unique sequence probe. It will be understood by those of skill in the art that not all of the amplified unique sequence elements are necessarily pooled after the amplification step. In a non-limiting example, in embodiments where the PCR reactions for each primer set are carried out separately, an aliquot from the PCR reaction can be analyzed, for example by gel electrophoresis, to determine whether the expected unique sequence element has been amplified. Those reactions that did not result in amplification of the desired unique sequence element are not used for pooling of the amplified unique sequence elements. However, using the methods of the invention, successful amplification of the unique sequence element without contaminating repetitive sequence elements was found more than 98% of the time, eliminating a requirement for such gel analysis. However, such methods may still be used for verification, as well as to provide an assessment of the amount of amplified unique sequence elements.

In the method of the invention those PCR reactions that resulted in amplification of the desired unique sequence element are subjected to a second round of PCR, using the same primer sets and PCR conditions, to produce larger quantities of the unique sequence element of interest, which are then pooled. Those of skill in the art will be aware that such further amplifications can be carried out more than once, and/or can be scaled up to produce larger amounts of the relevant unique sequence elements prior to pooling. Such further PCR reactions can be optionally evaluated by gel electrophoresis, as described above.

Approximately equal molar amounts of each secondary PCR product (as judged, for example, by intensity of the PCR products in an ethidium bromide stained agarose gel, or by appropriate spectrophotometric analysis) are then pooled. The pool of PCR products can then be optionally purified from residual un-extended primers using a purification column such as the QIAquick PCR Purification Kit or the MinELute 96 UF PCR Purification Kit (Qiagen, Valencia CA) or YM-30 microcon columns (Millipore), although this process is not required, but may provide for more accurate quantification of the PCR products.

Concentration and purity of the resulting unique sequence probe ("USP") can then be evaluated, for example, by spectrophotometrically measuring UV absorbance at 260 and 280nm and calculating the 260/280 ratio.

The USP can then be labeled with a detectable label. Useful detectable labels include but are not limited to radioactive labels such as ³²P, ³H, and ¹⁴C; fluorescent dyes such as fluorescein isothiocyanate (FITC), rhodamine, lanthanide phosphors, Texas red, and ALEXIS^{™} (Invitrogen/Molecular Probes), CY^{™} dyes (Amersham) and Spectrum dyes (Abbott Labs), electron-dense reagents such as gold; enzymes such as horseradish peroxidase, beta-galactosidase, luciferase, and alkaline phosphatase; colorimetric labels such as colloidal gold; magnetic labels such as those sold under the mark DYNABEADS^{™}; biotin; dioxigenin; or haptens and proteins for which antisera or monoclonal antibodies are available. In a most preferred embodiment, the USP is labeled with a fluorescent nucleoside analog, such as dUTP, in a direct labeling reaction using any number of common labeling techniques, such as the BioPrime Labeling kit (Invitrogen). In a further embodiment the USP can be labeled with a non-fluorescent moiety such as biotin, and then detected in subsequent post-hybridization assays using a fluorescent sandwiching technique such as avidin, followed by a fluorescent anti-avidin antibody technique

The label can be directly incorporated into the polynucleotide, or it can be attached to a molecule which hybridizes or binds to the polynucleotide. The labels may be coupled to the isolated polynucleotides by any means known to those of skill in the art. In a various embodiments, the isolated polynucleotides are labeled using nick translation, PCR, or random primer extension (see, e.g., Sambrook et al. *supra*). Methods for detecting the label include, but are not limited to spectroscopic, photochemical, biochemical, immunochemical, physical or chemical techniques.

The USP can be in lyophilized form, or in a solution comprising one or more of buffer solutions, hybridization solutions, and solutions for keeping the compositions in storage. Such a solution can be made as such, or the composition can be prepared at the time of use. Alternatively, the USP can be placed on a solid support, such as in a microarray, bead, nylon membrane, slide, or microplate format.

Unique sequence probes made by the methods of the invention, including but not limited to those disclosed herein can be used in any type of hybridization assay for a nucleic acid target of interest, including but not limited to in situ hybridization, fluorescent in situ hybridization, chromogenic in situ hybridization, and related techniques. The USP can be in lyophilized form, or in a solution comprising one or more of buffer solutions, hybridization solutions, and solutions for keeping the compositions in storage. Such a solution can be made as such, or the composition can be prepared at the time of use. Alternatively, the USP can be placed on a solid support, such as in a microarray, bead, nylon membrane, slide, or microplate format. In this alternative format, one skilled in the art will recognize that when the unique sequence probe is attached to a solid support and hybridized with a second detectable probe containing a repetitive sequence, competitor DNA will not be required in the hybridization because there will be no complementary repetitive sequences in the unique sequence probe attached to the solid support.

In a preferred embodiment, the unique sequence probe comprises or consists of a plurality of nucleic acids selected from the group consisting of **SEQ ID NOS: 893-986** (SMARCE1 USP).

In a further preferred embodiment, the unique sequence probe comprises or consists of a plurality of nucleic acids selected from the group consisting of **SEQ ID NOS: 987-1082** (PDCD6IP USP).

In a further preferred embodiment, the unique sequence probe comprises or consists of a plurality of nucleic acids selected from the group consisting of **SEQ ID NOS: 1083-1154** (CYP24 USP).

In a further preferred embodiment, the unique sequence probe comprises or consists of a plurality of nucleic acids selected from the group consisting of **SEQ ID NOS: 1155-1242** (NR1D1 USP).

In a further preferred embodiment, the unique sequence probe comprises or consists of a plurality of nucleic acids selected from the group consisting of **SEQ ID NOS: 1243-1338** (BIRC5 USP). It is further preferred that the unique sequence probe used in the invention comprises or consists of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, or 96 of the recited nucleic acids. Also used in the invention are polynucleotide primer sets, wherein a "polynucleotide primer set" comprises or consists of a forward and reverse polynucleotide primer ("primer pair") that are complementary to a given unique sequence element in a nucleic acid probe and can be used in a PCR to amplify a defined region of the given unique sequence element in the nucleic acid probe, wherein each polynucleotide primer in the primer set has an annealing temperature of between 52°C and 65°C; preferably between 55°C and 63°C, and more preferably between 56°C and 61°C, and wherein the polynucleotide primers in a polynucleotide primer set have an annealing temperature within 3°, 2°, 1°, or 0° C of each other.

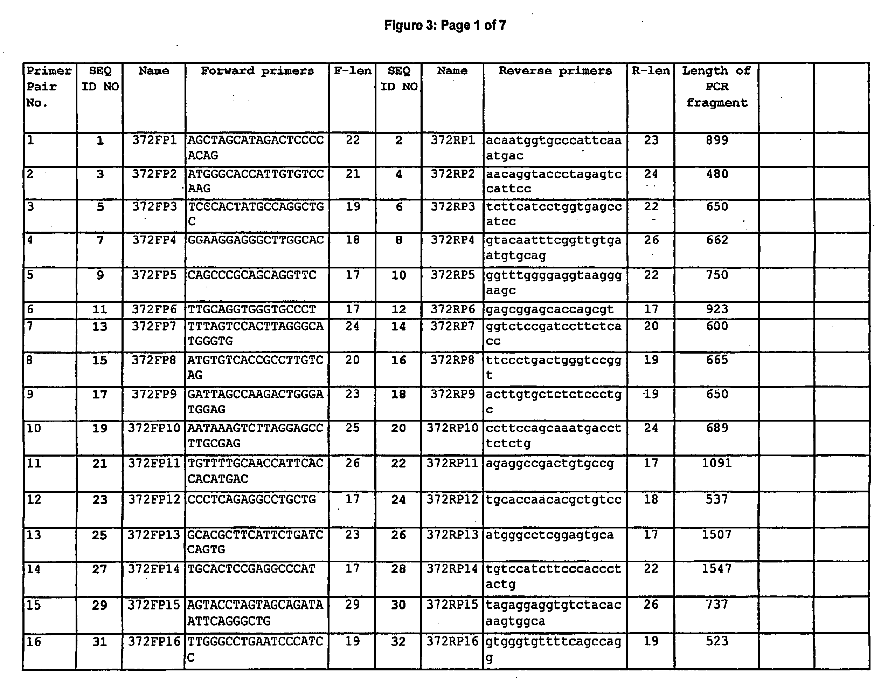

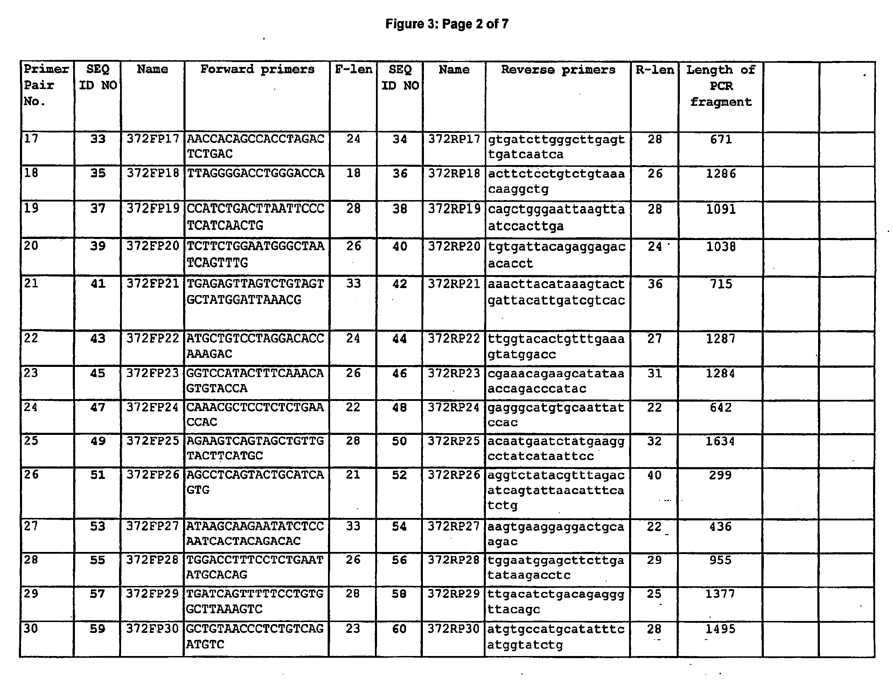

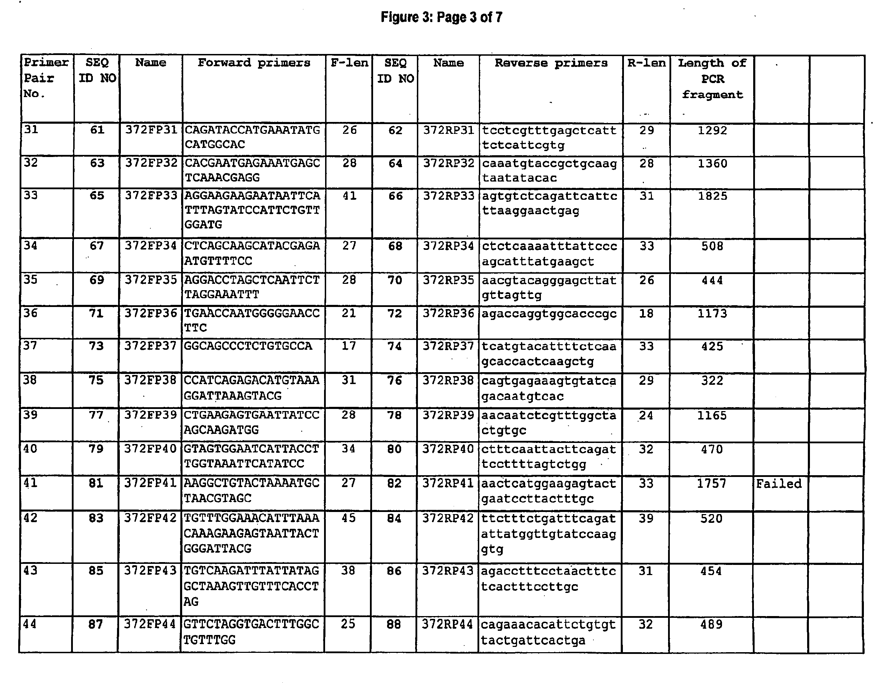

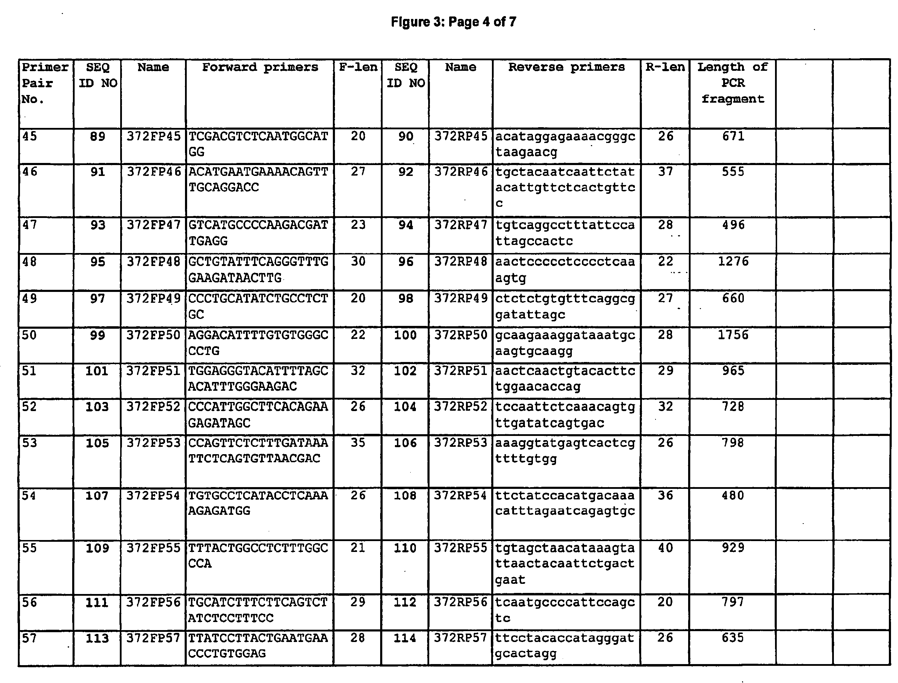

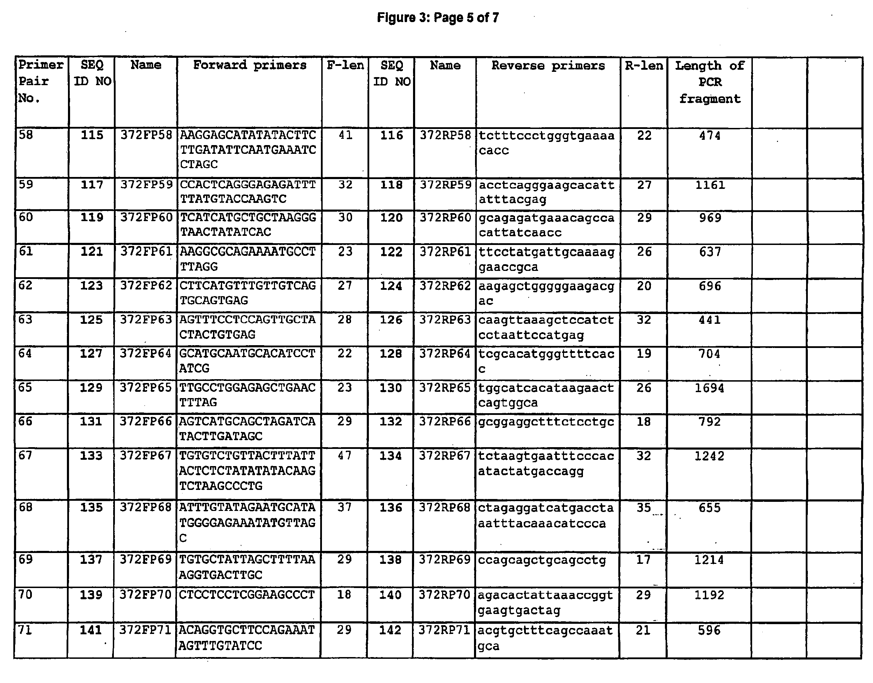

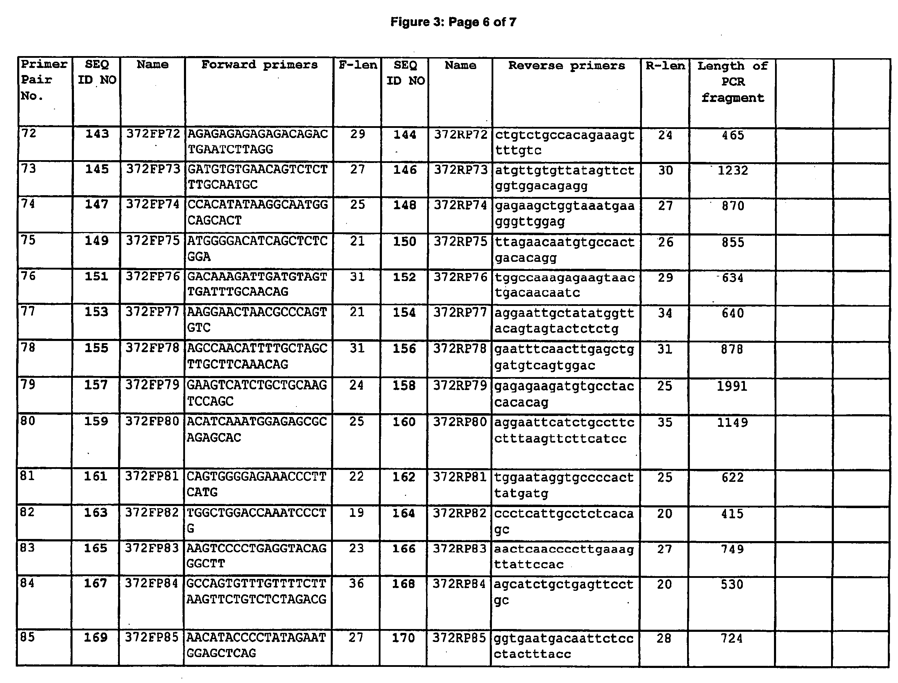

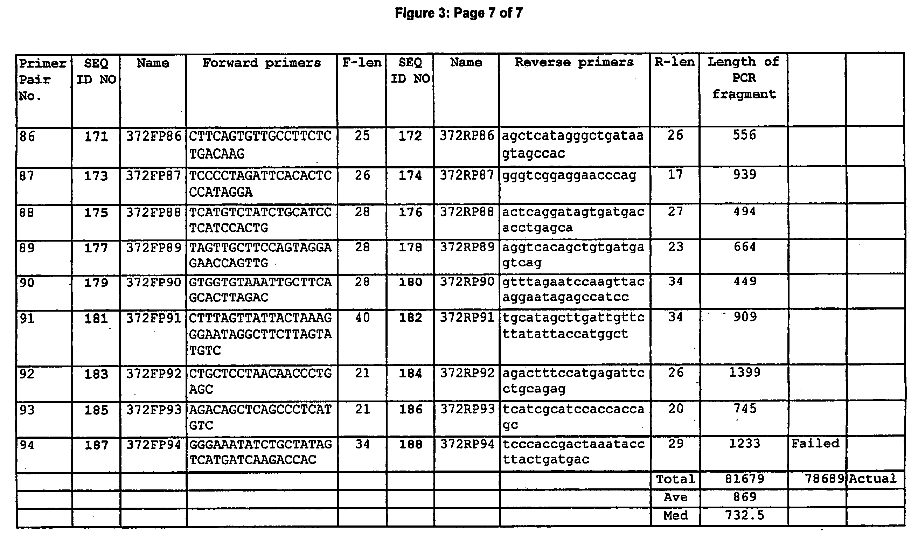

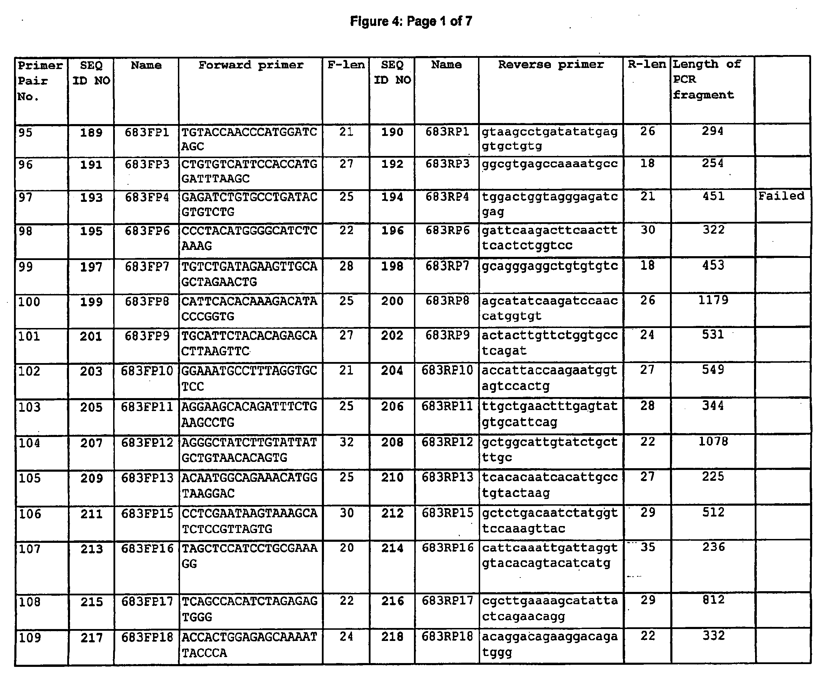

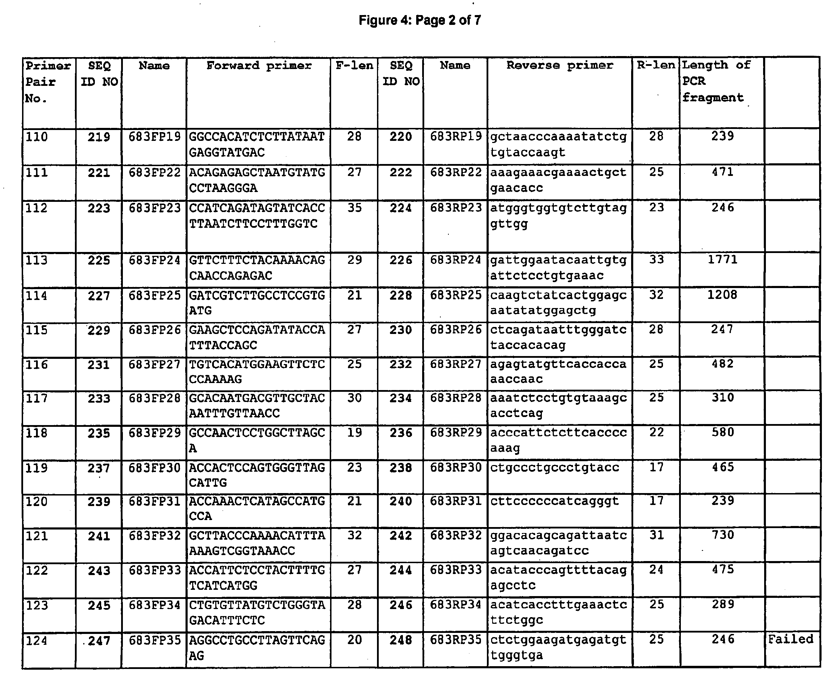

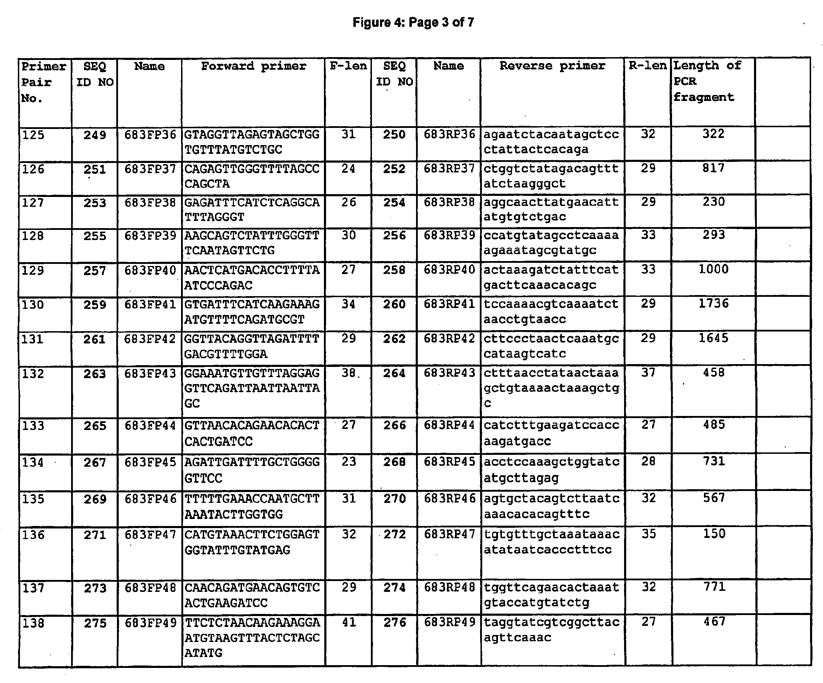

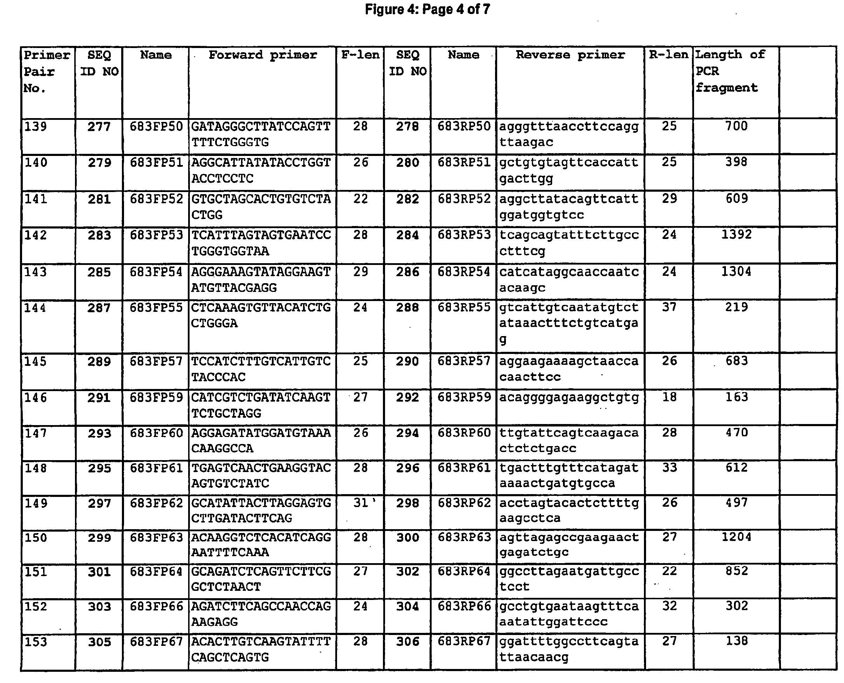

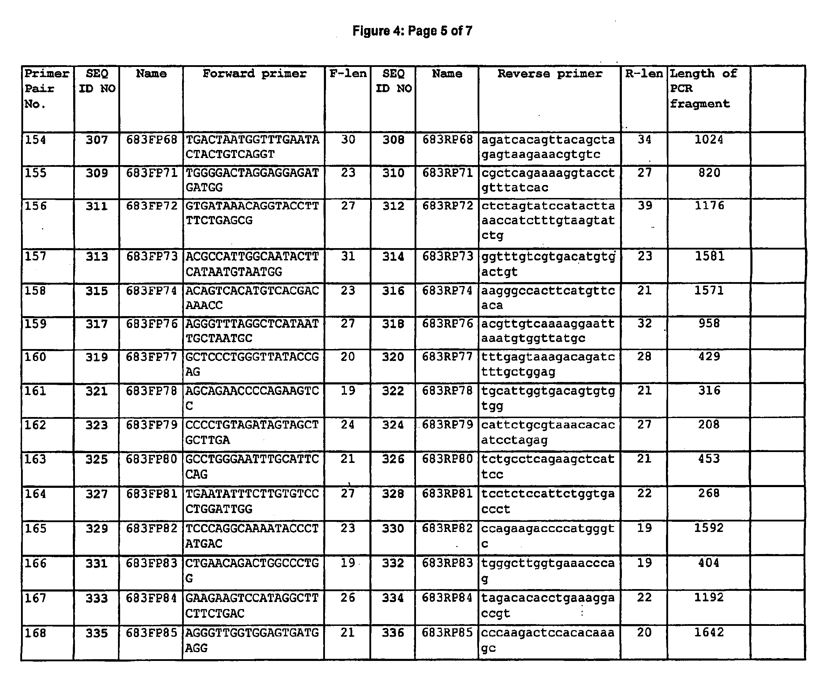

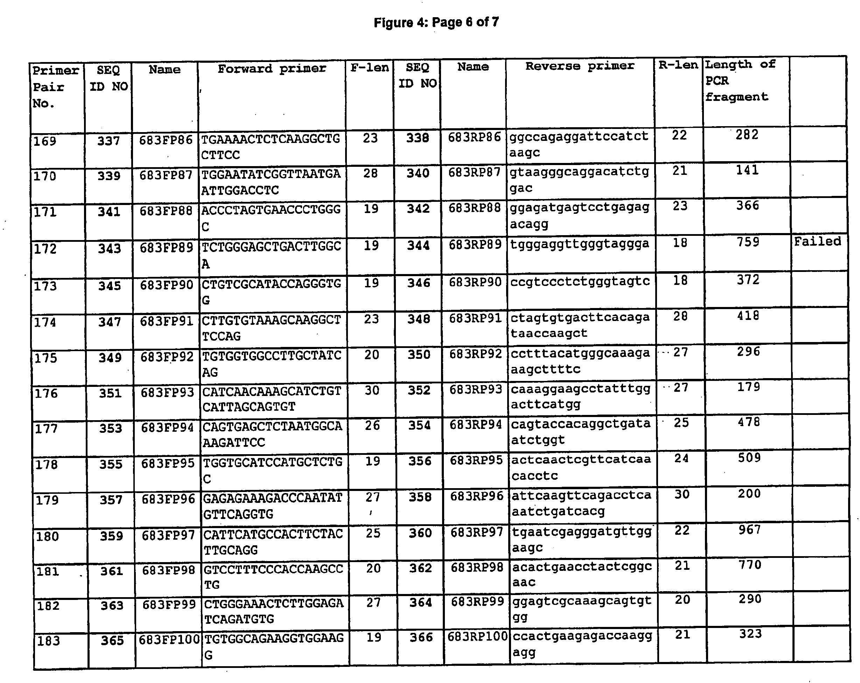

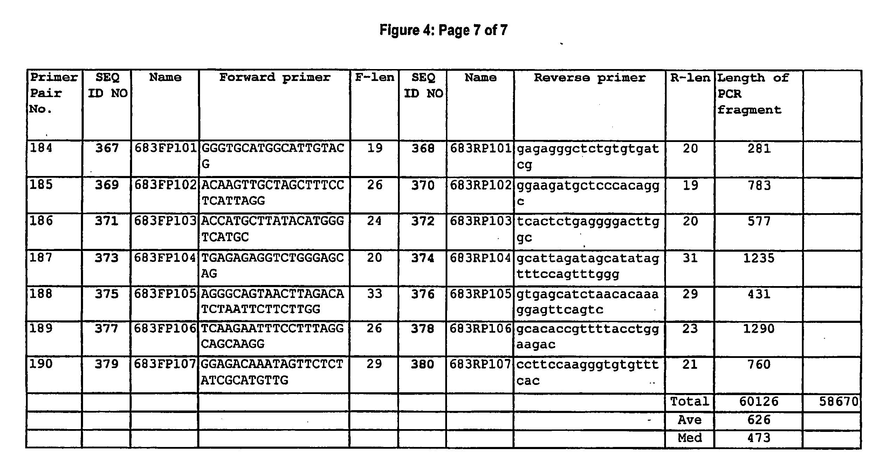

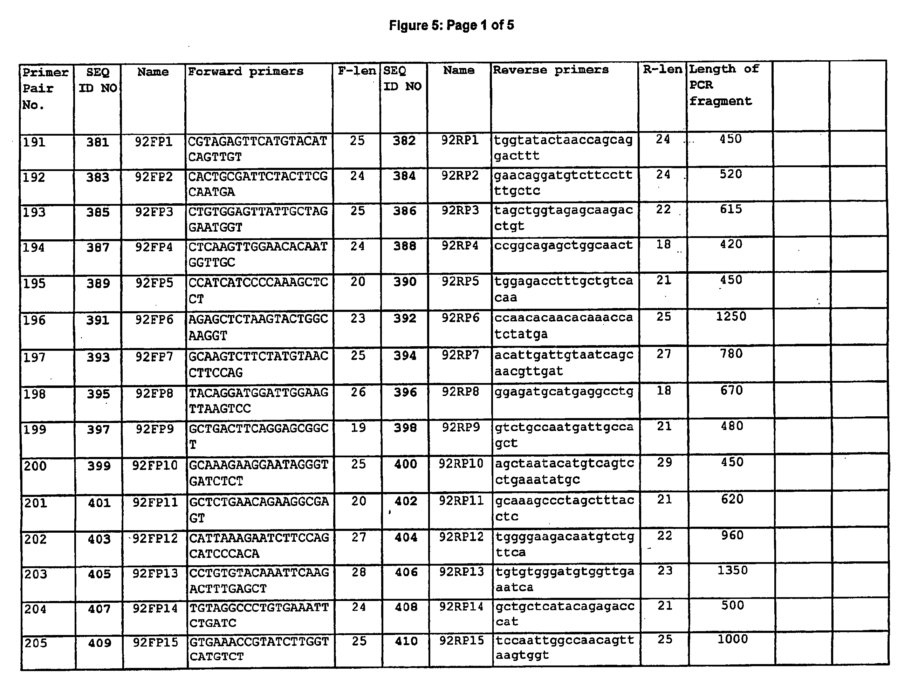

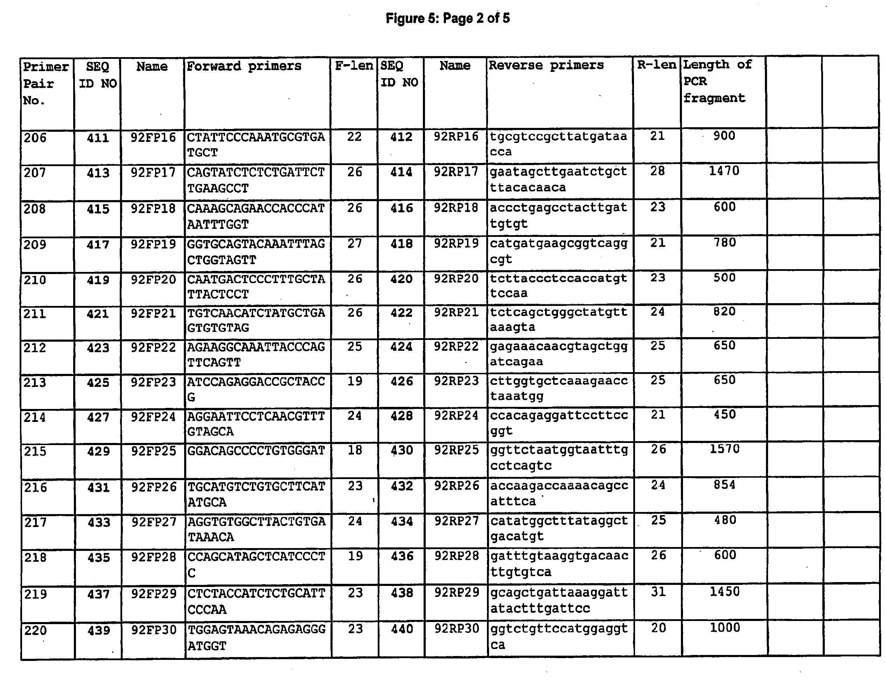

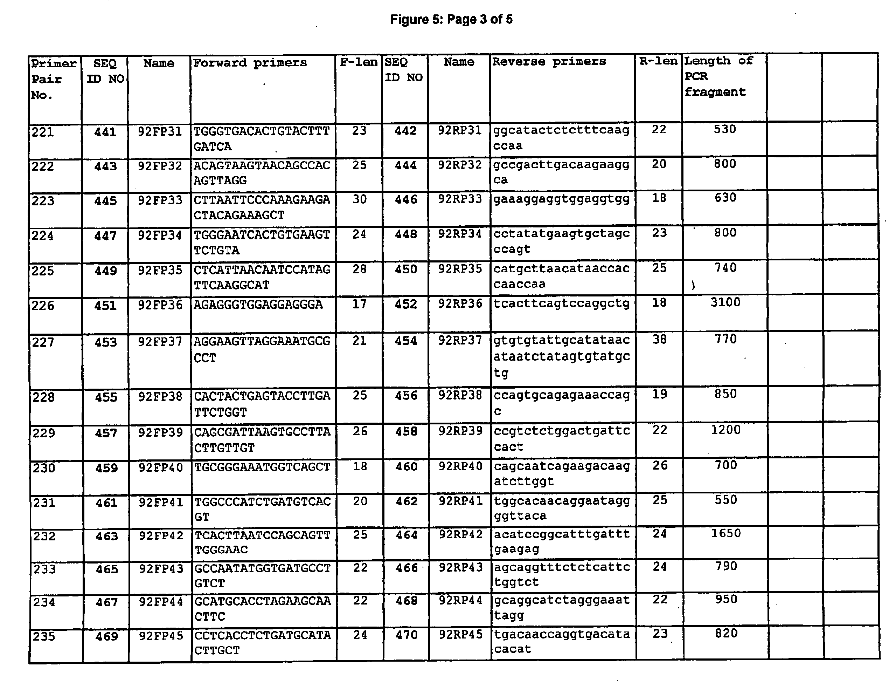

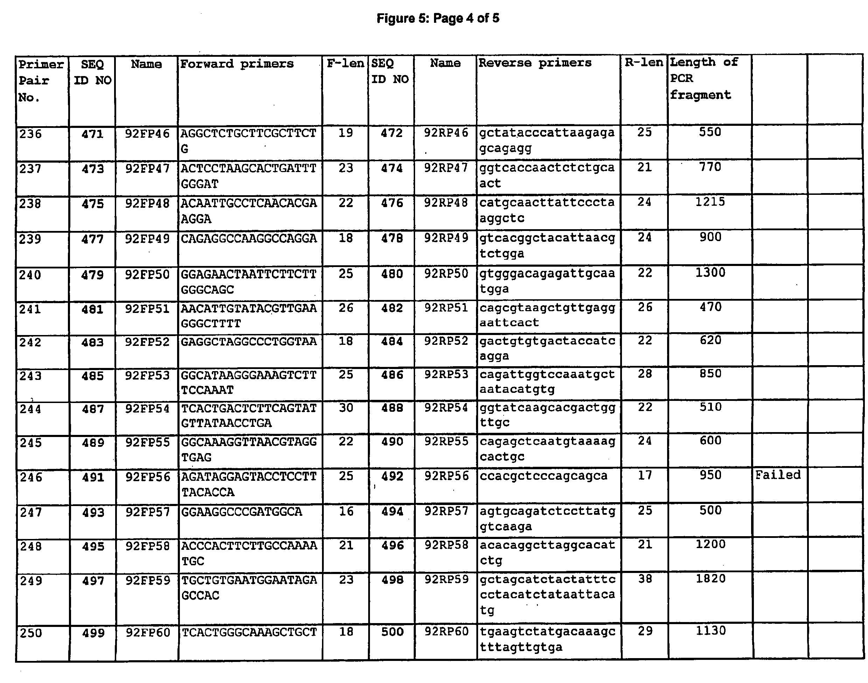

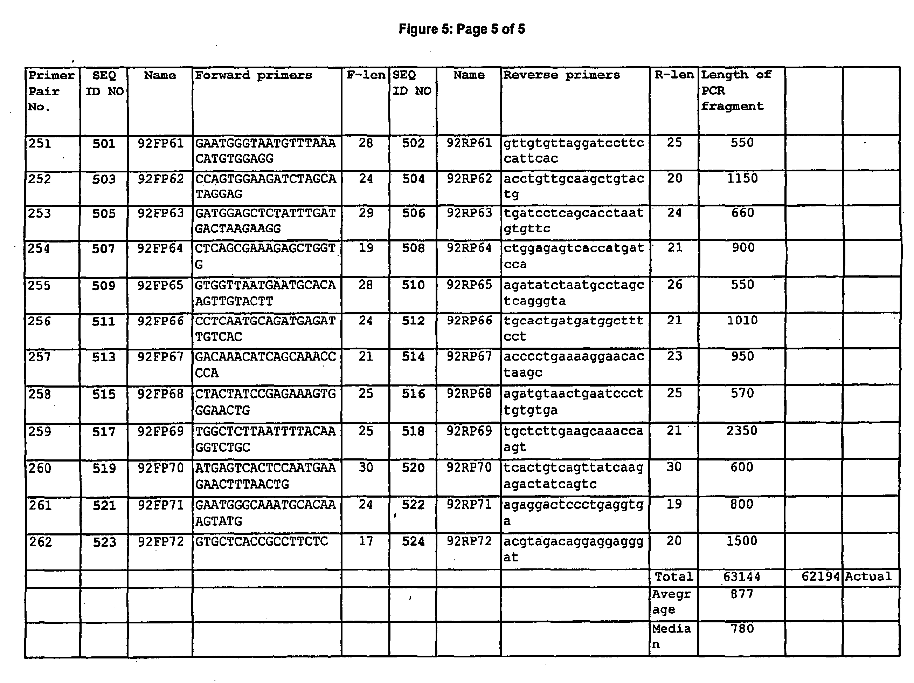

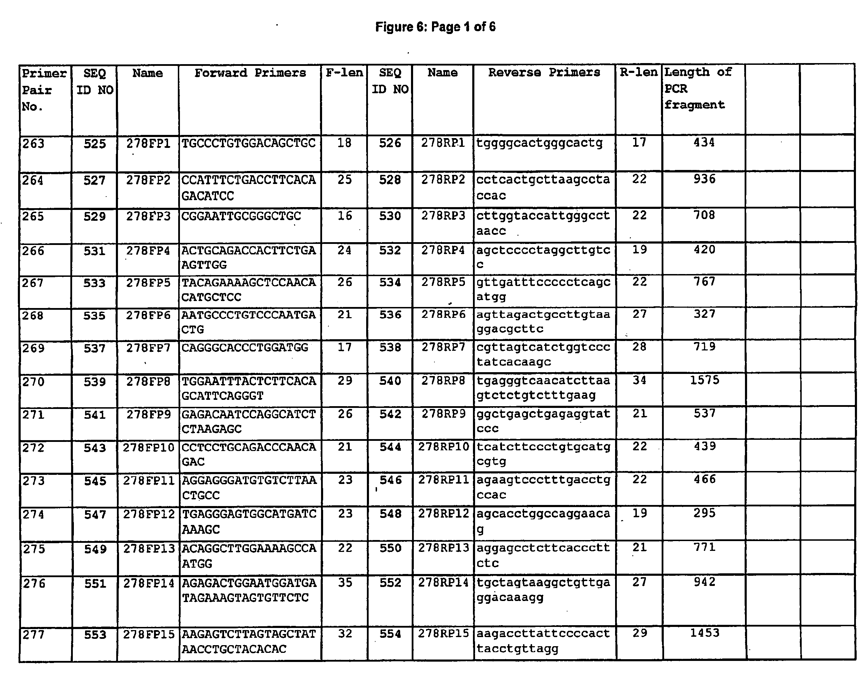

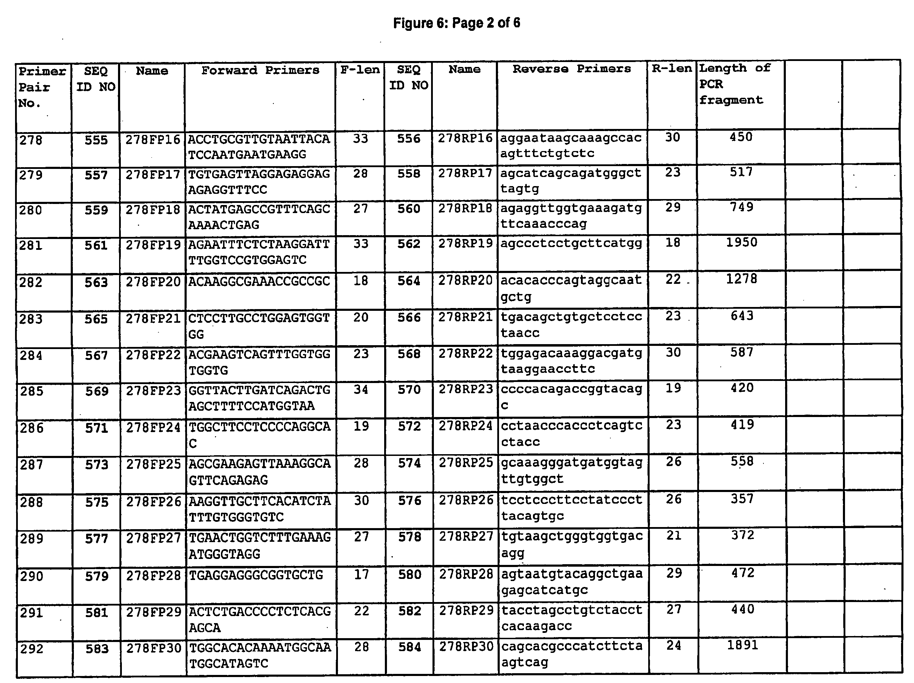

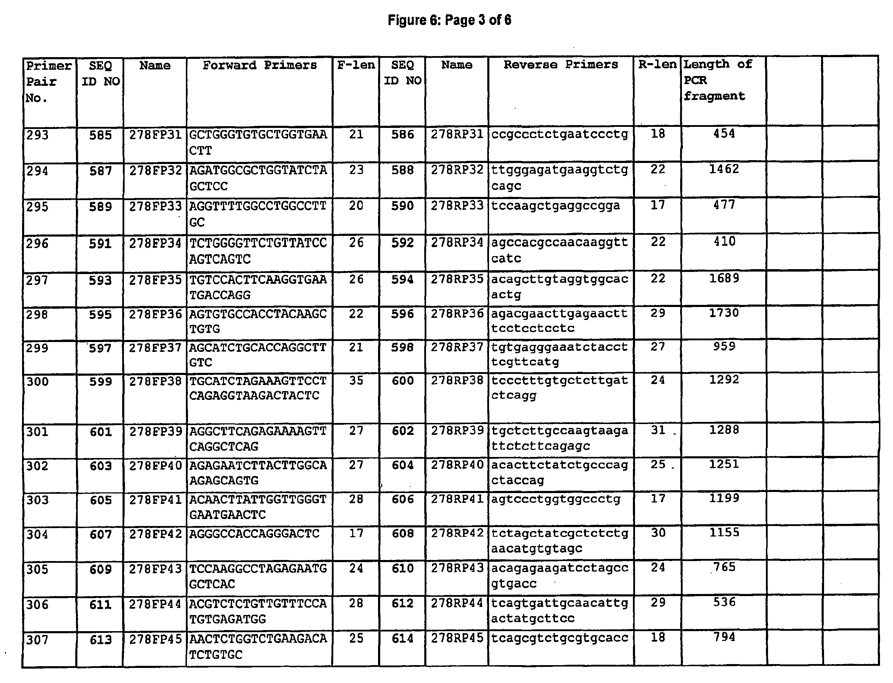

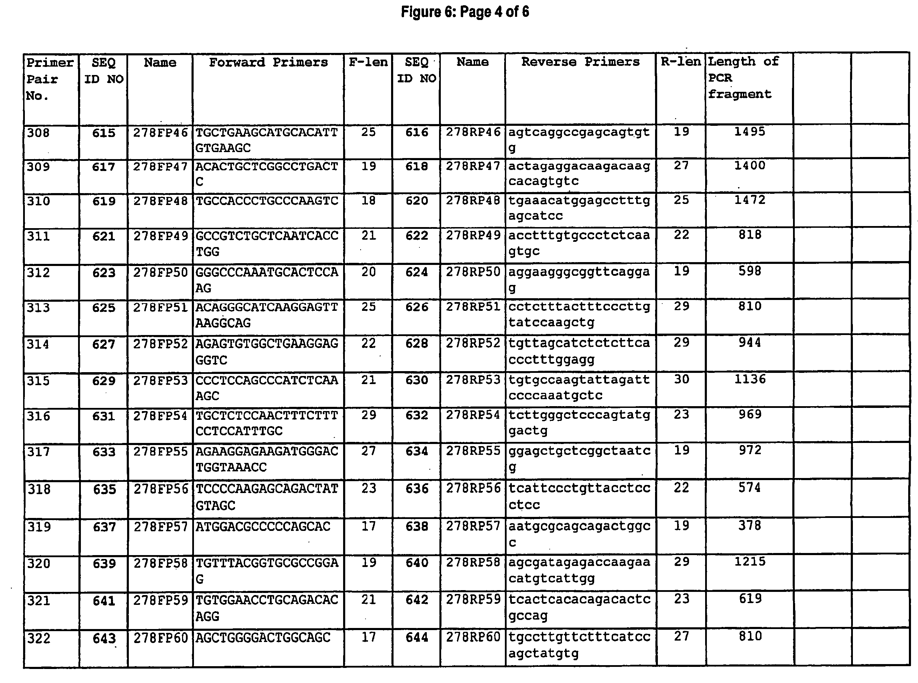

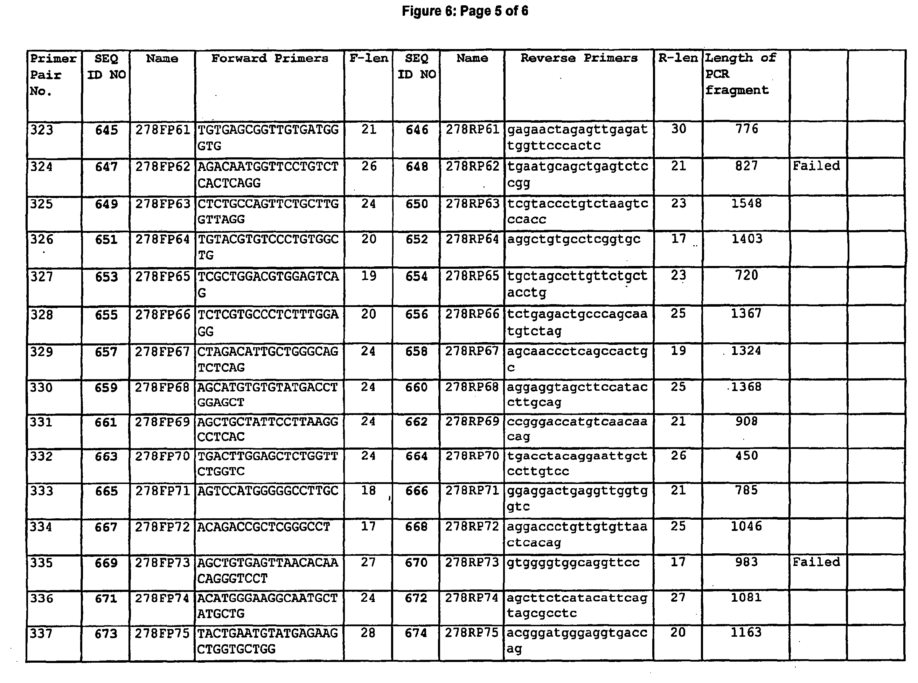

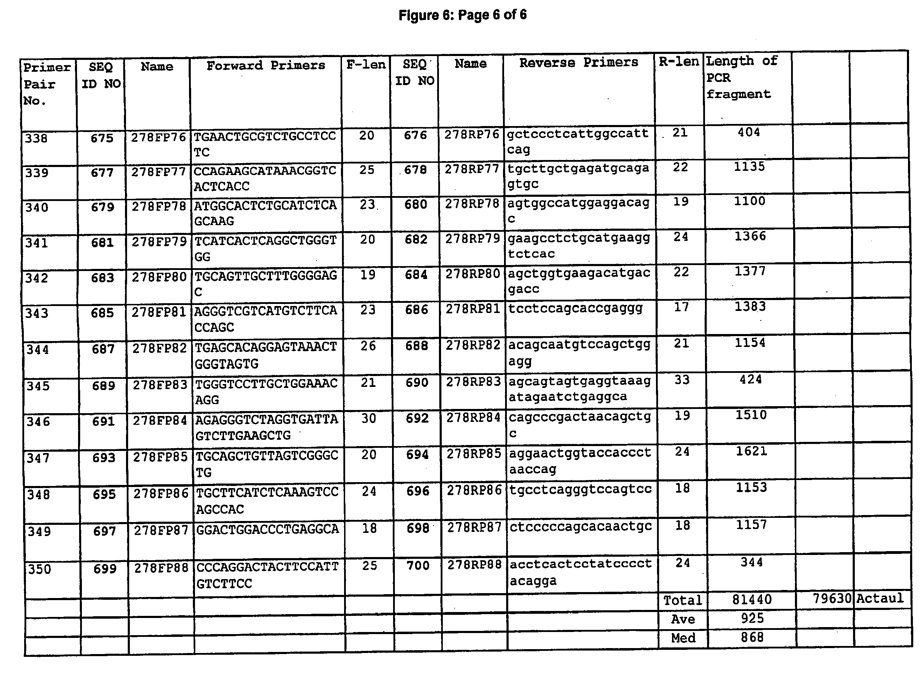

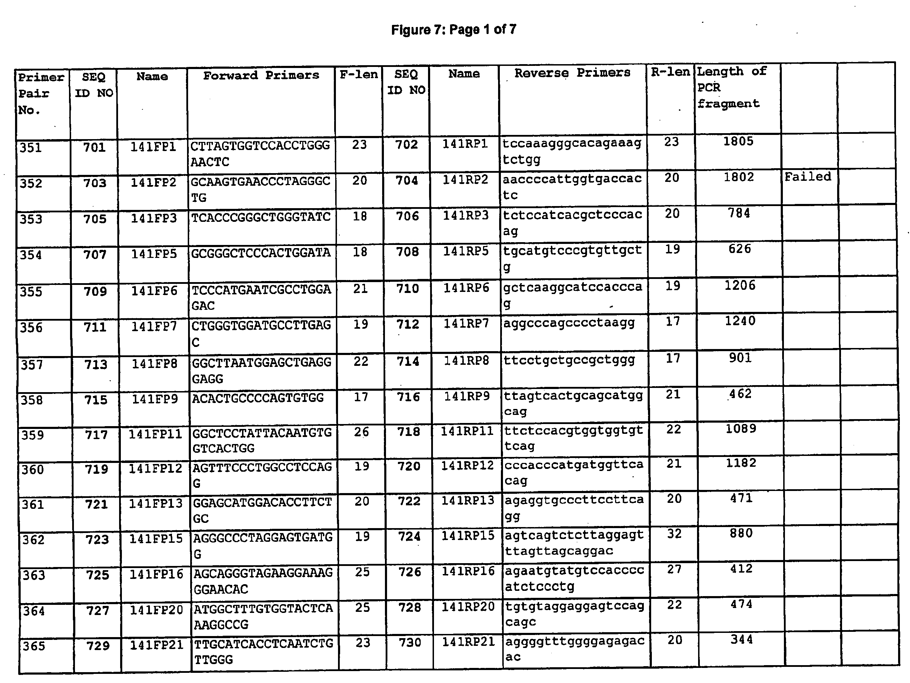

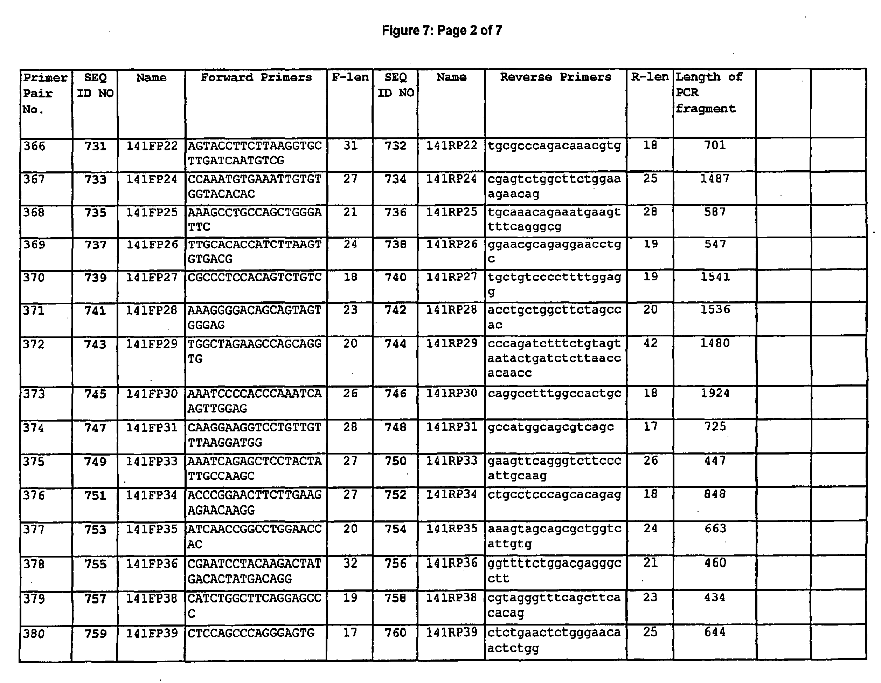

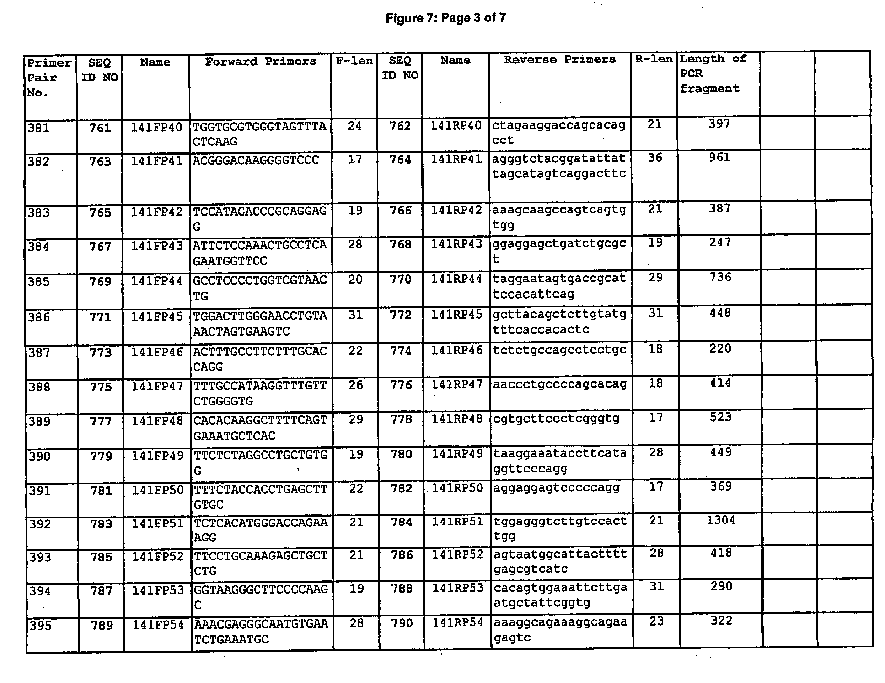

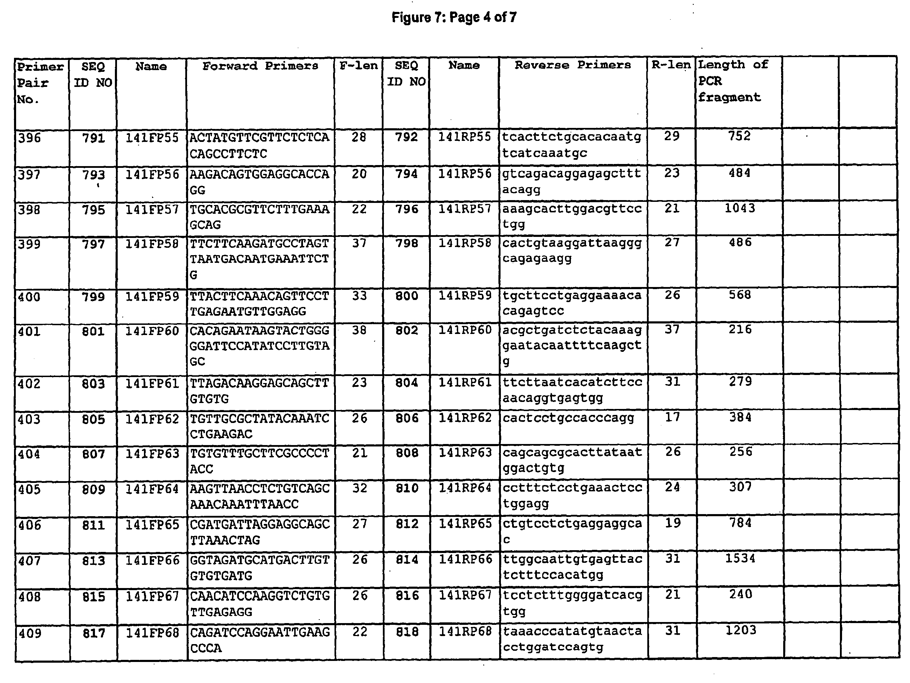

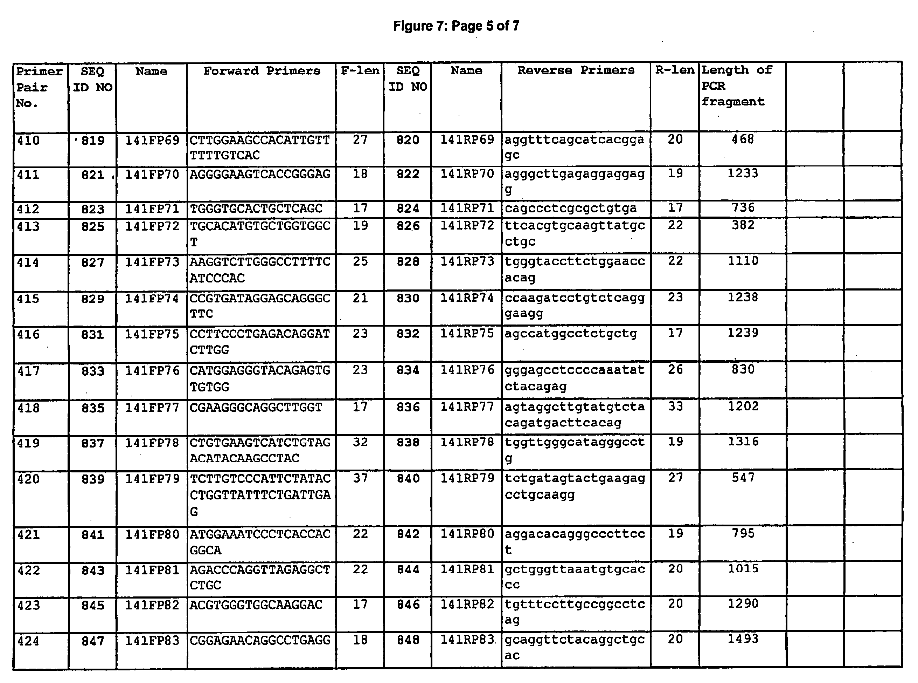

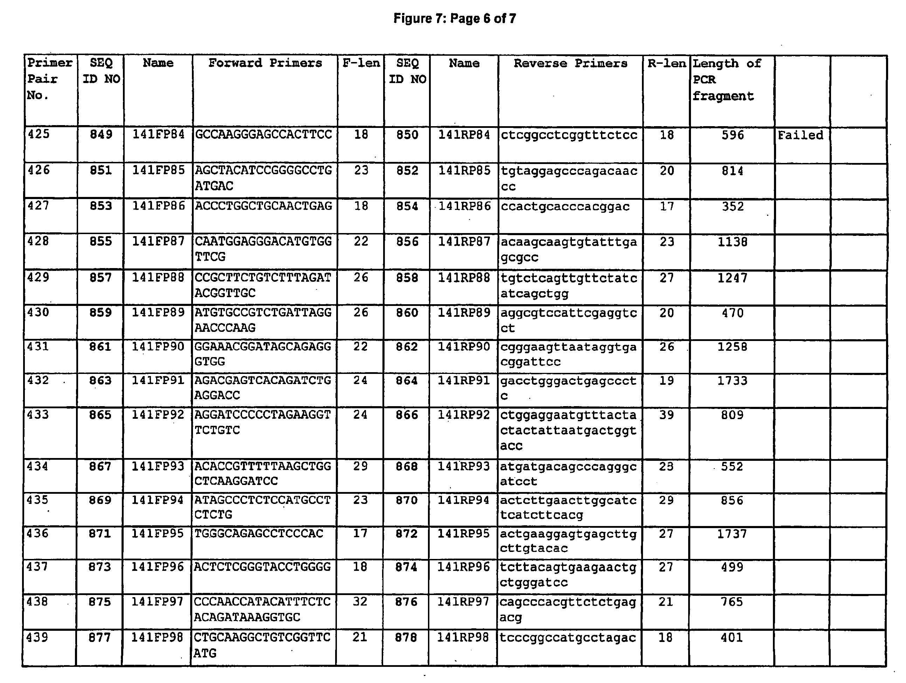

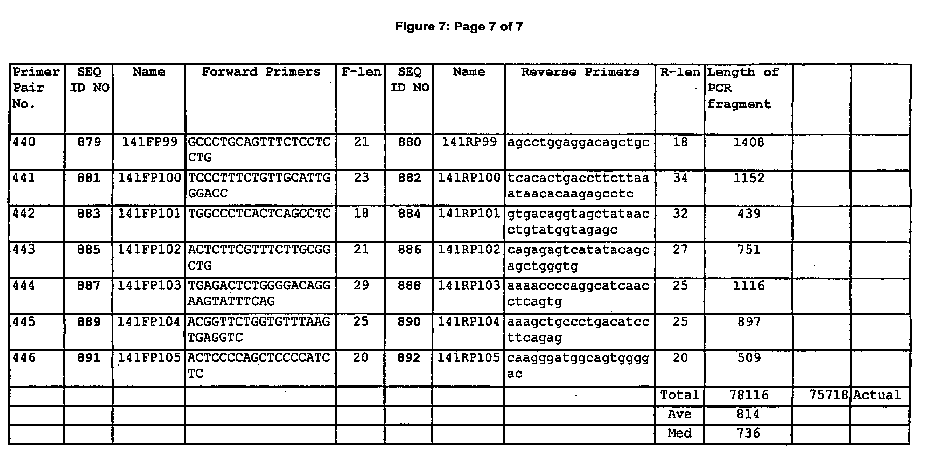

**Figures 3-7** provide a large number of such primer pairs for specific nucleic acid probes. In these Figures, the forward and reverse polynucleotide primers for a given polynucleotide primer set are listed on the same row in the Table and are numbered. For example, **SEQ ID NO:1** and **SEQ ID NO:2** in **Figure 3** constitute primer pair #1 that can be used, for example, to amplify a defined region of SMARCE1. Those of skill in the art will recognize that many such primer pairs are provided in **Figures 3-7****.**

In a prefered embodiment of the method, the polynucleotide primer set comprises or consists of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, or 96 of the primer pairs for a given nucleic acid probe (SMARCE1, PDCD6IP, CYP24, NR1D1, and BIRC5) selected from the group of primer pairs provided in **Figures 3-7****.**

In a further aspect, the present invention provides methods for detecting a nucleic acid target of interest, comprising:
(a) generating a unique sequence probe according to the methods of the invention for the nucleic acid target of interest;
(b) contacting the unique sequence probe to a specimen to be tested under conditions to promote hybridization of the unique sequence probe to the nucleic acid target; and
(c) detecting hybridization complexes formed between the unique sequence probe and the nucleic acid target, wherein such hybridization complexes provide a measure of the nucleic acid target in the specimen.

In this aspect, generating unique sequence probes is as described above in the first aspect of the invention. Nucleic acid targets are also as defined above.

As used herein "contacting" includes the step of denaturing the target nucleic acids in the specimen and the USP and bringing them into contact to facilitate hybridization. Any techniques known in the art can be used for such contacting. For example, while the contacting can take place prior to addition of the hybridization solution (for example, in a separate denaturing solution), it is preferred that both denaturation of the target nucleic acid and probe, and hybridization occurs in the same solution. While the contacting of the specimen with the hybridization solution can occur simultaneously with or prior to contacting of the specimen with the labeled USP, it is preferred that the labeled probes are added to the hybridization solution and that denaturation of the target nucleic acid and the probes occurs simultaneously in the hybridization solution.

In a preferred embodiment, the methods utilize hybridization buffers disclosed in US Patent Nos. 5,750,340 and 6,022,689, incorporated by reference herein in their entirety.

As used herein, the "specimen" refers to any specimen on which detection of a nucleic acid target by hybridization to the USP is useful, including but not limited to genomic DNA, total RNA, mRNA, cDNA, cell and tissue samples; surgical specimens, blood samples, bone marrow samples, cerebral spinal fluid, and metaphase chromosomal spreads. In a preferred embodiment, the specimen is a cell sample, tissue sample, or metaphase chromosomal spread. Methods for preparing such cell, tissue, and chromosomal spread samples are well known in the art. In a preferred embodiment, the specimen is fixed on a platform, such as any substrate upon which ISH can be performed, including but not limited to glass or plastic slides, and multiwell tissue culture plates, and the USP is labeled and used as a probe against the specimen.

Alternatively, the USP can be arrayed on a solid support and nucleic acids from the specimen can be labeled and used to probe the arrayed USP. In this embodiment, the USP is not labeled. In this embodiment, further, competitor DNA is not required to disable repetitive sequences found in the probe, because there are no complementary repetitive sequences in the unique sequence probe attached to the solid support.

Conditions to promote hybridization can be any such as are known in the art. Any conditions in which the USP binds selectively to the nucleic acid target to form a hybridization complex, and minimally or not at all to other sequences, can be used in the methods of the present invention. The exact conditions used will depend on the length of the unique sequence elements in the USP, their GC content, as well as various other factors as is well known to those of skill in the art. (See, for example, Tijssen (1993) Laboratory Techniques in Biochemistry and Molecular Biology-Hybridization with Nucleic Acid Probes part I, chapt 2, "Overview of principles of hybridization and the strategy of nucleic acid probe assays," Elsevier, N.Y. ("Tijssen"); and Fish: A Practical Approach, Barbara G. Beatty, Sabine Mai, and Jeremy A. Squire, editors. Oxford University Press, 2002.

Any desirable post-hybridization processing steps can be carried out. It is preferred that the specimens on the platform are dried, such as by air-drying, prior to detection. Optionally, the biological samples can be counterstained to detect cell structures, such as counterstaining with 4,6-diamidino-2-phenylindole (DAPI) or propidium iodide (PI) solution to stain nuclei.

Any method for detecting hybridization complexes can be used, such as by Southern blotting and Northern blotting methods, in situ hybridization, polymerase chain reaction (PCR) analysis, comparative genomic hybridization, or array based methods. In a preferred embodiment, detection is performed by in situ hybridization ("ISH"). In situ hybridization assays are well known to those of skill in the art. See, for example, US Patent Nos. 5,750,340 and 6,022,689, incorporated by reference herein in their entirety.

As discussed above, the labeled USP has a detectable moiety attached. The detectable moiety can be directly detectable, such as when a fluorescent-tagged nucleotide analog is covalently attached to the probe. Alternatively, the attached moiety can be detected in a secondary detection procedure, such as when a fluorescent tagged antibody specific to the attached moiety is added after the hybridization and wash procedures. Methods for detecting the label present in a hybridization complex include, but are not limited to spectroscopic, photochemical, biochemical, immunochemical, physical or chemical techniques. For example, useful labels include but are not limited to radioactive labels such as ³²P, ³H, and ¹⁴C; fluorescent dyes such as fluorescein isothiocyanate (FITC), rhodamine, lanthanide phosphors, and Texas red, Spectrum Dyes (Abbott Labs), ALEXIS^{™} (Molecular Probes), CY^{™} dyes (Amersham); electron-dense reagents such as gold; enzymes such as horseradish peroxidase, beta-galactosidase, luciferase, and alkaline phosphatase; colorimetric labels such as colloidal gold; magnetic labels such as those sold under the mark DYNABEADS^{™}; biotin; dioxigenin; or haptens and proteins for which antisera or monoclonal antibodies are available. The label can be directly incorporated into the polynucleotide, or it can be attached to a probe or antibody which hybridizes or binds to the polynucleotide. The labels may be coupled to the probes by any means known to those of skill in the art. In a various embodiments, the probes are labeled using nick translation, PCR, TAQMAN^{™}, or random primer extension (see, e.g., Sambrook et al. *supra*).

Signals from the labeled probes can be detected by any means known in the art for the particular label employed. For example, where the detectable label is a fluorescent label, one can detect fluorescence signals by visualization with a fluorescence microscope.

### Examples:

The complete DNA sequence containing the genomic region of interest is obtained. Clones containing the sequence of interest can include but are not limited to cDNA clones, lambda, cosmid, or clones, or large insert clones such as YACs, BACs or P1s. Complete DNA sequences of clones can be obtained using a variety of manual or automated DNA sequencing protocols. The following describes the procedure for obtaining the unique sequence of, for example, BAC clones. A common source for sequences of BAC clones can be obtained at the UCSC Genome Bioinformatics database (http://genome.ucsc.edu/index.html?org=Human). The public database can also be used as a source of DNA sequence information for any genomic region of interest. The repetitive elements of the BACs can be identified using the "Mask Repeat" function at the UCSC Genome Bioinformatics database, and repeat-free sequences of the BACs can then obtained using the "Get DNA" function. PCR primers flanking each unique sequence component are then designed, using either a manual approach or a computational approach. Several software programs area available for primer design, both commercially and in web-based format, such as "Fast PCR" (http://www.biocenter.helsinki.fi/bi/bare-1_html/oligos.htm). These programs calculate the melting temperatures and identify potential problems such as self annealing hairpin loop formation and primer-dimerization. For a typical BAC, the number of primer pairs for amplification required to amplify the entire unique sequence component of the clones ranges from 50 to 100 or more.

To demonstrate the present invention, 5 BAC DNAs were selected. The BACs contain the genes for NR1D1, SMARCE1, BIRC5, CYP24A, and PDCD6IP. The BACs were selected from the "32K human genome BAC Rearray", maintained at the CHORI (http://bacpac.chori.org/). The sizes of the BACS in this example range from 154-178 kb. Names, sizes, and chromosome locations of the individual BACs are summarized in **Table 1.**

**Table 1**

| Clone | Size of BAC (kb) | Chromosome Location |
|---|---|---|
| PDCD6IP | 150.4 | 3p23 |
| SMARCE1 | 180.8 | 17q21.2 |
| BIRC5 | 177.6 | 17q25.3 |
| NR1D1 | 162.0 | 17q21.1 |
| CYP24 | 180.9 | 20q13.2 |

The repetitive elements of the BACs were identified using the "Mask Repeat" function at the UCSC Genome Bioinformatics database, and repeat-free sequences of the BACs were obtained using the "Get DNA" function (http://genome.ucsc.edu/index.html?org=Human). The sequence of the related BAC clone for each marker was down loaded from Human UCSC Genome browser with the repetitive sequences marked. Primers were selected from both ends of a unique sequence area of no less than 300 bp length. Primers were designed for each BAC using the "Fast PCR" program.

The selected primers were as follows (used in primer pairs as noted in **Figures 3-7**):
a) SMARCE1 primers: **SEQ ID NOS:1-188;**
b) PDCD6IP primers: **SEQ ID NOS: 189-380;**
c) CYP24 primers: **SEQ ID NOS: 381-524;**
d) NR1D1 primers: **SEQ ID NOS: 525-700;** and
e) BIRC5 primers: **SEQ ID NOS: 701-892.**

PCR was carried out as follows:
- PCR Master Mix from Promega (Catalog # M7505) was used for PCR. Reaction buffer: 25 units /ml of Taq DNA Polymerase, in manufacturer's proprietary reaction buffer -(pH 8.5), 200 uM dATP, 200 uM dGTP, 200 uM dCTP, 200 uM dTTP, 1.5 mM MgCl₂.
- Cycling conditions:
   1. 5 minutes at 95°C;
   2. 15 seconds at 95°C;
   3. 30 seconds at 56°C; (This temperature varies depending on the annealing temperature of the primer.)
   4. 2 minutes at 72°C;
   5. go to step 2 for 30 times;
   6. 3 minutes at 72°C;
- Primer concentration was 1 uM.
- Template concentration:

- The BAC clone concentration for the first run PCR was between 0.3 and 0.5 ng/ul. For the second run PCR (see below for: "second run PCR"), 1/100 volume of the first run PCR reaction mix was used as template and concentration was not determined.
- For a 50 ul first run PCR reaction, mix:
   0.3 ul BAC template (56 ng/ul);
   5 ul forward primer (10 uM);
   5 ul reverse primer (10 uM);
   25 ul 2X PCR master mix;
   14.7 ul sterile dH₂O
- And run through the above cycle and store at -20°C.
- For a 50 ul second run PCR reaction, mix
   0.5 ul first run PCR product;
   5 ul forward primer (10 uM);
   5 ul reverse primer (10 uM);
   25 ul 2X PCR master mix;
   14.5 ul sterile dH₂O
- And run through the above cycle and store at -20°C

The melting temperature for all the primers used ranged from 57° to 60°C. Analytical agarose gels demonstrating amplified PCR products showed that 98-99% of the reactions were successful.

The numbers of primers pairs for each BAC, the average length of the PCR products, and the total coverage of the original BAC included in the unique sequence probe are summarized in Table 2.

**Table 2**

| Clone | Complete sequence of clone (bp) | Unique sequence in clone(bp) | % unique sequence in clone | Number of PCR primer pairs | Average size of PCR product s (bp) | Amplified sequence (bp) | % complete sequence in clone amplified | % unique sequence amplified |
|---|---|---|---|---|---|---|---|---|
| PDCD61P | 150437 | 77036 | 51.2 | 96 | 1240 | 59000 | 39.2 | 76.6 |
| SMARCE1 | 180862 | 105440 | 58.3 | 94 | 1720 | 79000 | 43.7 | 74.9 |
| BIRC5 | 177623 | 97050 | 54.6 | 96 | 814 | 76000 | 42.8 | 78.3 |
| NR1D1 | 161994 | 95161 | 58.7 | 88 | 925 | 80000 | 49.4 | 84.1 |
| CYP24 | 180862 | 105440 | 58.3 | 72 | 877 | 62000 | 34.3 | 58.8 |

Sequences of the resulting PCR products are provided as follows:
(a) SMARCE 1 USP: **SEQ ID NOS: 893-986**
(b) PDCD6IP USP: **SEQ ID NOS: 987-1082**
(c) CYP24 USP: **SEQ ID NOS:1083-1154**
(d) NR1D1 USP: **SEQ ID NOS: 1155-1242;** and
(e) BIRC5 USP: **SEQ ID NOS: 1243-1338.**

The PCR products were purified from the un-extended primers and pooled, and then aliquots of each pool were labeled with at least one of four fluorochromes: Spectrum Orange, Spectrum Green, Spectrum Red (Vysis) or DEAC (diethylaminocoumarin-5-dUTP, PerkinElmer) using Invitrogen's BioPrime DNA Labeling Kit, according to manufacturer's instructions. Unincorporated fluorochromes were purified using YM-30 microcon columns (Millipore).

Probes were hybridized either alone, in pairs, or in triplets (ie: for 1, 2, or 3 nucleic acid targets; and thus 1, 2, or 3 different USPs) to metaphase chromosomes or to breast cancer thin sections, or to sectioned tissue culture cell lines sectioned from paraffin blocks. The hybridization buffer was 20% formamide, 10% dextransulfate, 0.9% NaCl. The probes concentrations were 40 ng/µl if labeled with Spectrum Orange, Texas Red, or DEAC Aqua, and 60 ng/µl for the Spectrum Green. The specimens were hybridized at 38°C for 14-20 hours. After hybridization, the slides were washed with 0.15% NaCl at 60°C for 10 minutes.

For each example, the original BAC and its derivative USP were labeled with one of the four fluorochromes co-hybridized on metaphase chromosomes. In each pair of dual labeled probes. Competitor DNA was included in these co-hybridizations in order to suppress the repetitive sequences contained in the parental BACs. In each of the pairs, the probes co-hybridize with equal intensity to the expected chromosome region, without hybridization to any other chromosome region, and without any specific or non specific background or artifactual hybridization to any other chromosome regions. These dual hybridizations demonstrate that there is no loss of specificity or signal quality between the labeled parental BAC and its derivative USP.

On metaphase chromosomes, the signal intensities for all probes were equal for all five probes, regardless of the fluorochrome. In all cases bright, strong signals were clearly visible on the correct chromosome, without any artifactual signal on other chromosomes. There was no background hybridization randomly distributed throughout the genome, nor were there any areas of diffuse non-specific hybridization.

Multiplex hybridization of probe triplets was also tested on 5uM formalin-fixed, paraffin embedded, interphase breast adenocarcinoma cell line thin sections. In these hybridizations, clear distinct hybridization signals were seen, in the three unique colors expected for the fluorochromes used, without artifactual signals or interfering background. Thus there is no loss of signal quality or intensity whether the probes were hybridized singly or in triplets.

Probe quality and signal strength were not dependent on particular pairs of probes and fluorochromes. Pair-wise combinations of fluorochromes and probes that have been tested are summarized in **Table 3.**

**Table 3 summarizes the combinations of probes with fluorochromes that have been examined. Signals quality is rated ++++ if strong clear unambiguous without background or artifactual signals.**

| Clone | Spectrum Orange | Texas Red | Spectrum Green | DEAC Aqua |
|---|---|---|---|---|
| NR1D1 | ++++ | | | |
| SMARCE1 | | ++++ | ++++ | |
| BIRC5 | ++++ | | ++++ | ++++ |
| CYP24 | ++++ | ++++ | ++++ | ++++ |
| PDCD6IP | ++++ | | ++++ | |

## Claims

1. A method for preparing unique sequence probes comprising:
(a) identifying unique sequence elements contained in a nucleic acid probe, wherein the nucleic acid probe comprises at least 10 unique sequence elements and a plurality of repetitive sequence elements, and wherein the at least 10 unique sequence elements in the nucleic acid probe selectively bind to a nucleic acid target of interest;
(b) amplifying at least 10 unique sequence elements in the nucleic acid probe to generate at least 10 amplified unique sequence elements, wherein the amplifying comprises
(i) contacting the nucleic acid probe with at least 10 primer sets for the at least 10 unique sequence elements under conditions to promote hybridization of the at least 10 primer sets to the nucleic acid probe, wherein each primer in the at least 10 primer sets has an annealing temperature of between 52° C and 65° C, and wherein the annealing temperature for all of the primers in the at least 10 primer sets are within 3° C of each other; and
(ii) amplifying the at least 10 unique sequence elements defined by the at least 10 primer sets by polymerase chain reaction under conditions that comprise annealing the at least 10 primer sets to the nucleic acid probe at a temperature between 51° C and 65° C;
(c) subjecting those PCR reactions that resulted in amplification of the desired unique sequence element to a second round of PCR, using the same primer sets and PCR conditions; and
(d) pooling the amplified unique sequence elements to produce the unique sequence probe.

2. The method of claim 1 wherein the contacting comprises contacting the nucleic acid probe with at least 50 primer sets for at least 50 unique sequence elements in the nucleic acid probe, and wherein the amplifying unique sequence elements comprises amplifying the at least 50 unique sequence elements.

3. The method of claim 1 further comprising labeling the unique sequence probe.

4. The method of claim 1 wherein the contacting of the nucleic acid probe with the primer sets comprises separately contacting each individual primer set with the nucleic acid probe, and the amplifying comprises amplifying each unique sequence element in a separate polymerase chain reaction.

5. The method of claim 4 wherein the separate polymerase chain reactions are conducted simultaneously.

6. The method of claim 1, further comprising placing the unique sequence probe on a solid support.

7. A method for detecting a nucleic acid target of interest, comprising:
(a) generating a unique sequence probe according to the method of claim 1;
(b) contacting the unique sequence probe to a specimen to be tested under conditions to promote hybridization of the unique sequence probe to the nucleic acid target; and
(c) detecting hybridization complexes formed between the unique sequence probe and the nucleic acid target, wherein such hybridization complexes provide a measure of the nucleic acid target in the specimen.

## Patentansprüche

1. Verfahren zur Herstellung einzigartiger Sequenzsonden, umfassend:
(a) Bestimmen einzigartiger Sequenzelemente, die in einer Nukleinsäuresonde enthalten sind, wobei die Nukleinsäuresonde mindestens 10 einzigartige Sequenzelemente und eine Vielzahl repetitiver Sequenzelemente umfasst, und wobei die mindestens 10 einzigartigen Sequenzelemente in der Nukleinsäuresonde selektiv an eine interessierende Zielnukleinsäure binden;
(b) Vervielfältigen von mindestens 10 einzigartigen Sequenzelementen in der Nukleinsäuresonde, um mindestens 10 vervielfältigte einzigartige Sequenzelemente zu erzeugen, wobei die Vervielfältigung umfasst
(i) In-Kontakt-Bringen der Nukleinsäuresonde mit mindestens 10 Primersets für die mindestens 10 einzigartigen Sequenzelemente unter Bedingungen, die das Hybridisieren der mindestens 10 Primersets an die Nukleinsäuresonde begünstigen, wobei jeder Primer in den mindestens 10 Primersets eine Annealingtemperatur von zwischen 52°C und 65°C aufweist und wobei die Annealingtemperaturen für all diese Primer in den mindestens 10 Primersets innerhalb von 3°C zueinander liegen; und
(ii) Vervielfältigung der mindestens 10 einzigartigen Sequenzelemente, die durch die mindestens 10 Primersets definiert sind, mittels Polymerasekettenreaktion unter Bedingungen, die das Annealing der mindestens 10 Primersets an die Nukleinsäuresonde bei einer Temperatur zwischen 51°C und 65°C umfassen;
(c) Unterziehen derjenigen PCR-Reaktionen, welche zu einer Vervielfältigung der gewünschten einzigartigen Sequenzelemente führten, einer zweiten PCR-Runde unter Verwendung derselben Primersets und PCR-Bedingungen; und
(d) Vereinigen der vervielfältigten einzigartigen Sequenzelemente, um die einzigartige Sequenzsonde herzustellen.

2. Verfahren nach Anspruch 1, wobei das In-Kontakt-Bringen das In-Kontakt-Bringen der Nukleinsäuresonde mit mindestens 50 Primersets für mindestens 50 einzigartige Sequenzelemente in der Nukleinsäuresonde umfasst und wobei die Vervielfältigung der einzigartigen Sequenzelemente die Vervielfältigung der mindestens 50 einzigartigen Sequenzelemente umfasst.

3. Verfahren nach Anspruch 1, zusätzlich umfassend ein Markieren der einzigartigen Sequenzsonde.

4. Verfahren nach Anspruch 1, wobei das In-Kontakt-Bringen der Nukleinsäuresonde mit den Primersets ein separates In-Kontakt-Bringen jedes einzelnen Primersets mit der Nukleinsäuresonde umfasst und wobei das Vervielfältigen das Vervielfältigen jedes einzigartigen Sequenzelements in einer separaten Polymerasekettenreaktion umfasst.

5. Verfahren nach Anspruch 4, wobei die separaten Polymerasekettenreaktionen gleichzeitig durchgeführt werden.

6. Verfahren nach Anspruch 1, zusätzlich umfassend das Aufbringen der einzigartigen Sequenzsonde auf einen festen Träger.

7. Verfahren zum Nachweis einer interessierenden Zielnukleinsäure, umfassend:
(a) Herstellung einer einzigartigen Sequenzsonde gemäß dem Verfahren nach Anspruch 1;
(b) In-Kontakt-Bringen der einzigartigen Sequenzsonde mit einer Probe, die getestet werden soll, unter Bedingungen, die die Hybridisierung der einzigartigen Sequenzsonde an die Zielnukleinsäure fördern; und
(c) Nachweis von Hybridisierungskomplexen, die zwischen der einzigartigen Sequenzsonde und der Zielnukleinsäure ausgebildet wurden, wobei solche Hybridisierungskomplexe ein Maß der Zielnukleinsäure in der Probe zur Verfügung stellen.

## Revendications

1. Procédé pour la préparation de sondes uniséquentielles, comprenant:
(a) l'identification d'éléments uniséquentiels contenus dans un acide nucléique sonde, tandis que l'acide nucléique sonde comprend au moins 10 éléments uniséquentiels et une pluralité d'éléments à séquences répétitives, et tandis que les au moins 10 éléments uniséquentiels dans l'acide nucléique sonde se lient sélectivement à un acide nucléique cible d'intérêt;
(b) l'amplification d'au moins 10 éléments uniséquentiels dans l'acide nucléique sonde pour générer au moins 10 éléments uniséquentiels amplifiés, tandis que l'amplification comprend
(i) la mise en contact de l'acide nucléique sonde avec au moins 10 ensembles d'amorces pour les au moins 10 éléments uniséquentiels dans des conditions destinées à favoriser l'hybridation des au moins 10 ensembles d'amorces avec l'acide nucléique sonde, tandis que chaque amorce dans les au moins 10 ensembles d'amorces a une température d'annelage entre 52°C et 65°C, et tandis que les températures d'annelage pour la totalité des amorces dans les au moins 10 ensembles d'amorces diffèrent entre elles d'au plus 3°C; et
(ii) l'amplification des au moins 10 éléments uniséquentiels définis par les au moins 10 ensembles d'amorces par réaction d'amplification en chaîne par polymérase dans des conditions qui comprennent l'annelage des au moins 10 ensembles d'amorces avec l'acide nucléique sonde à une température entre 51°C et 65°C;
(c) l'application aux réactions d'ACP qui conduisaient à l'amplification de l'élément uniséquentiel désiré d'un deuxième cycle d'ACP, avec utilisation des mêmes ensembles d'amorces et des mêmes conditions d'ACP; et
(d) le regroupement des éléments uniséquentiels amplifiés pour produire la sonde uniséquentielle.

2. Procédé selon la revendication 1, dans lequel la mise en contact comprend la mise en contact de l'acide nucléique sonde avec au moins 50 ensembles d'amorces pour au moins 50 éléments uniséquentiels dans l'acide nucléique sonde, et dans lequel l'amplification d'éléments uniséquentiels comprend l'amplification des au moins 50 éléments uniséquentiels.

3. Procédé selon la revendication 1, comprenant en outre le marquage de la sonde uniséquentielle.

4. Procédé selon la revendication 1, dans lequel la mise en contact de l'acide nucléique sonde avec les ensembles d'amorces comprend la mise en contact séparément de chaque ensemble d'amorces individuel avec l'acide nucléique sonde, et l'amplification comprend l'amplification de chaque élément uniséquentiel dans une réaction d'amplification en chaîne par polymérase séparée.

5. Procédé selon la revendication 4, dans lequel les réactions d'amplification en chaîne par polymérase séparées sont conduites simultanément.

6. Procédé selon la revendication 1, comprenant en outre le placement de la sonde uniséquentielle sur un support solide.

7. Procédé pour la détection d'un acide nucléique cible d'intérêt, comprenant:
(a) la génération d'une sonde uniséquentielle conformément au procédé selon la revendication 1;
(b) la mise en contact de la sonde uniséquentielle avec un spécimen à tester dans des conditions destinées à favoriser l'hybridation de la sonde uniséquentielle avec l'acide nucléique cible; et
(c) la détection de complexes d'hybridation formés entre la sonde uniséquentielle et l'acide nucléique cible, tandis que de tels complexes d'hybridation procurent une mesure de l'acide nucléique cible dans le spécimen.
